(19) Europäisches Patentamt
European Patent Office
Office européen des brevets

(11) **EP 4 166 668 A1**

(12) **EUROPEAN PATENT APPLICATION**
published in accordance with Art. 153(4) EPC

(43) Date of publication:
**19.04.2023 Bulletin 2023/16**

(21) Application number: **21823138.9**

(22) Date of filing: **06.05.2021**

(51) International Patent Classification (IPC):
**C12N 15/113** (2010.01)    **A61K 31/713** (2006.01)
**A61P 35/00** (2006.01)

(52) Cooperative Patent Classification (CPC):
**A61K 31/713; A61P 35/00; C12N 15/113**

(86) International application number:
**PCT/CN2021/091834**

(87) International publication number:
**WO 2021/249064 (16.12.2021 Gazette 2021/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.06.2020 CN 202010524139**

(71) Applicant: **Ractigen Therapeutics
Nantong, Jiangsu 226400 (CN)**

(72) Inventors:
• **LI, Longcheng
Nantong, Jiangsu 226400 (CN)**
• **KANG, Moorim
Nantong, Jiangsu 226400 (CN)**

(74) Representative: **Høiberg P/S
Adelgade 12
1304 Copenhagen K (DK)**

Remarks:
The complete document including Reference
Table(s) and the Sequence Listing(s) can be
downloaded from the EPO website

(54) **NUCLEIC ACID MOLECULE TARGETING MITF GENE AND USE THEREOF**

(57)    Provided is a double-stranded nucleic acid molecule, in particular to a small activating nucleic acid molecule, wherein the small activating nucleic acid molecule may be a nucleic acid molecule targeting MITF gene and at least comprises a first oligonucleotide strand. Further provided are compositions or formulations comprising the small activating nucleic acid molecule and uses thereof. The small activating nucleic acid molecule, or the composition or formulation comprising the small activating nucleic acid molecule, can be used to activate or upregulate the MITF gene expression in a cell, and treat a disease or condition related to insufficient or decreased MITF protein expression.

FIG. 2

**Description**

**TECHNICAL FIELD**

The present invention relates to the technical field of nucleic acids, particularly relates to a nucleic acid molecule with a double-stranded structure related to gene activation, such as a small activating nucleic acid molecule, more particularly relates to a nucleic acid molecule targeting MITF gene, and also relates to use of the small activating nucleic acid molecule to activate / upregulate microphthalmia-associated transcription factor (MITF) in gene transcription. The present invention generally provides a compound, a pharmaceutical composition and a method of the use thereof, and more particularly, the present invention will be used in the treatment of a disease that benefits from increased MITF gene expression, such as vitiligo.

**BACKGROUND**

Vitiligo is an acquired hypopigmentation skin disease characterized by local reduction of epidermal and hair melanocytes and then the formation of irregular white patches. Statistically, patients with vitiligo account for about 0.5% to 2% of the global population, with no apparent gender difference (Picardo et al., 2015). Vitiligo can be divided into vitiligo vulgaris and segmental vitiligo according to clinical features, wherein the vitiligo vulgaris is related to the melanocyte impairment caused by autoimmune regulation or oxidative stress, and the segmental vitiligo is related to genetic factors (Gauthier, Cario Andre, and Taieb 2003; Dell'Anna and Picardo 2006).

Vitiligo is related to factors such as familial hereditary, autoimmune regulation, oxidative stress and functional melanocyte impairment. The occurrence of vitiligo has a distinct family aggregation tendency, the family history positive rate of the vitiligo patients is about 6.25% to 40%, the risk of developing vitiligo of the first-degree relatives is 18 folds that of the general population, and patients with a positive family history usually develop vitiligo earlier (Sehgal and Srivastava 2007). *In vitro* experiments show that a large amount of proinflammatory factors can inhibit the synthesis of melanin and the expression of transcription factors related to the melanin synthesis due to the increase of cytokines (IL-6, TNF-$\alpha$, IL-1$\beta$ and IL-8) produced by the immunoreaction in patients with vitiligo, (Kotobuki et al., 2012). Anti-melanocyte antibodies are detectable in the serum of 50% to 93% of patients with vitiligo and these antibodies can cause the reduction or disappearance of melanocytes through immune complex activation complement and/or antibody-dependent cell-mediated cytotoxicity (Kemp et al., 2007). Dell'Anna laboratory suggests that imbalance in oxidative stress in vitiligo patients may result from abnormal cardiolipin in epidermal cells, which can cause a decrease in mitochondrial electron transport chain activity, induce excessive production of reactive oxygenspecies (ROS), and lead to melanocyte death (Dell'Anna et al., 2007).

The loss of melanocytes is a key factor for the development of vitiligo, and the melanocytes release antigens after physical and chemical damage, stimulate an organism to produce an anti-melanocyte antibody, lead to the inactivation of a large number of melanocytes and aggravate epidermal depigmentation. Experiments have shown that melanin in skin lesions is damaged or lost as it traverses the epidermis, possibly due to adhesion limitations of melanocytes to the basement membrane and keratinocytes (Kumar, Parsad, and Kanwar 2011). Melanocyte dysfunction is mainly manifested by decreased number of melanocytes, dysfunction of adhesion and migration of melanocytes, excessive apoptosis of melanocytes, abnormal development and differentiation of melanocytes, and the like.

The microphthalmia-associated transcription factor encoded by MITF gene is a transcription factor having a basic-helix-loop-helix-leucine zipper (bHLH-Zip) structure and binding to DNA in the form of a dimer. MITF plays a key role in the development of melanocytes, retinal pigment epithelial cells, osteoclasts and mast cells. MITF together with the relevant factors including transcription factor EB (TFEB), TFE3 and TFEC constitutes the MiT family (Hemesath et al., 1994). MITF is the only factor in the MiT family that plays an important role in normal melanocyte development (Levy, Khaled, and Fisher 2006). MITF is not only a necessary regulatory factor for development, proliferation and survival of melanocytes, but also has a crucial role in regulating expression of related enzymes and melanosome proteins. Promoters of three major pigmentation-related enzymes tyrosinase, TYRP1 and DCT contain the universal binding site for MITF (TCATGTG), which is the target gene for MITF transcriptional regulation (Bentley, Eisen, and Goding 1994).

It has been found that MITF mutations can cause a variety of human diseases, including Waardenburg syndrome type 2A (WS2A), Tietze syndrome and COMMAD syndrome, which all exhibit severe deafness and pigmentation disorders, with the former two appearing as autosomal dominant inheritance.

At present, although there are various methods for treating vitiligo, there is no method for thoroughly treating vitiligo. Simple appearance improvement can be ensured by cosmetically masking white patches, and other treatments include steroid hormones, ultraviolet irradiation, surgery, and the like. Ultraviolet-B irradiation can blur the boundaries of white patches, however, phototherapy is at risk of carcinogenesis. In the early stage, Kwinter et al. used topical application of highly potent glucocorticoids to treat 70 patients with vitiligo in children with effective rate being up to 64% (Kwinter et al., 2007) while high recurrence rate. The psoralen-like drugs are clinically commonly used as photosensitizers to perform psoralen plus ultraviolet A photochemotherapy (PUVA) on vitiligo patients in cooperation with long-wave ultraviolet rays so as to improve the synthesis and anti-apoptosis effects of melanocytes at skin lesions (Iannella et al., 2016). Afamelanotide is the only drug approved by the U.S. Food and Drug Administration (FDA) for preclinical testing, but Afamelanotide is required to be tested in combination with narrow-band ultraviolet B phototherapy (NB-UVB). When narrow-band ultraviolet B phototherapy (NB-UVB) is required to activate melanocortin receptor 1 (MCIR), Afamelanotide binds to MCIR to produce melanin (Lim et al., 2015; Grimes et al., 2013), but the specific NB-UVB action duration and drug dosage require further experimentation. The surgical treatments including autologous scar epidermal transplantation, autologous micro-transplantation, autologous melanocyte transplantation, mixed epidermal transplantation, micro-staining and the like also have good curative effects, but these surgical operations have strict contraindications and the treatment method is limited.

Although each of the treatment methods has certain effect, the symptoms are easy to relapse, the treatment methods have great side effects, and effective frugal dose needs to be further determined, thereby bringing great limitation to clinical treatment.

## SUMMARY

In order to solve problems of the above one or more aspects, the present invention provides a small activating nucleic acid (small activating RNA, saRNA) molecule based on an RNA activation process for treating a disease or condition caused by MITF protein deficiency or insufficiency or by MITF single allelic mutations, such as vitiligo, Waardenburg syndrome type 2A or Tietze syndrome, by activating / upregulating MITF gene expression.

One objective of the present invention is to provide a small activating nucleic acid molecule based on an RNA activation process, which increases the MITF protein expression level by activating / upregulating MITF gene transcription, or use of the small activating nucleic acid molecule to prepare a drug for treating a disease or condition related to MITF protein deficiency or insufficiency.

Another objective of the present invention is to provide a composition or formulation comprising the small activating nucleic acid molecule.

Yet another objective of the present invention is to provide use of the small activating nucleic acid molecule or the composition or formulation comprising the same to prepare a drug for activating / upregulating the MITF gene expression in a cell.

Yet another objective of the present invention is to provide a method for activating / upregulating the MITF gene expression in a cell.

Yet another objective of the present invention is to provide a method for activating / upregulating the MITF gene expression in a cell, which can be used for treating a disease caused by MITF single allelic mutation, such as Waardenburg syndrome type 2A or Tietze syndrome.

Still another objective of the present invention is to provide use of the small activating nucleic acid molecule or the composition or formulation comprising the same to prepare a drug for treating a disease or condition related to MITF protein deficiency or insufficiency, such as vitiligo.

Another objective of the present invention is to provide an isolated small activating nucleic acid molecule target site of MITF gene, wherein the target site comprises a sequence of any 16 to 35 contiguous nucleotides of any sequence selected from SEQ ID NO: 299 to SEQ ID NO: 305, or a sequence having at least 75%, such as at least about 79%, about 80%, about 85%, about 90%, about 95%, about 99% or 100% homology to the sequence consisting of any 16 to 35 contiguous nucleotides.

In one aspect of the present invention, provided is a small activating nucleic acid molecule such as a small activating RNA (saRNA) molecule, the small activating nucleic acid molecule at least comprises a first oligonucleotide strand having at least 75% homology or complementarity to any consecutive fragment of 16 to 35 nucleotides in length in a promoter region of human MITF gene. Further, the promoter region of the human MITF gene is preferably a region of 500 nucleotides in length from a transcription start site (TSS) of the MITF gene to the upstream thereof (SEQ ID NO: 1), and the first oligonucleotide strand has at least 75% homology or complementarity to any consecutive fragment of 16 to 35 nucleotides in length in the region of 500 nucleotides in length from the transcription start site (TSS) of the MITF gene to the upstream thereof (SEQ ID NO: 1).

In one aspect of the present invention, provided is a small activating nucleic acid molecule, such as a small activating RNA (saRNA) molecule, which activates or upregulates the MITF gene expression in a cell. One strand of the small activating nucleic acid molecule has at least 75% homology or complementarity to any nucleic acid sequence of 16 to 35 nucleotides in length in a promoter region of MITF gene, thereby activating or upregulating the MITF gene expression, wherein the promoter region comprises 500 nucleotides (SEQ ID NO: 1) upstream of a transcription start site. Specifically, one strand of the small activating nucleic acid molecule comprises or is selected from a nucleic acid sequence having at least 75%, e.g., at least about 79%, about 80%, about 85%, about 90%, about 95%, about 99% or 100% homology or complementarity to a consecutive sequence of 16 to 35 nucleotides in length in positions -500 to -408 (region H1, SEQ ID NO: 299), -403 to -351 (region H2, SEQ ID NO: 300), -342 to -262 (region H3, SEQ ID NO: 301), -181 to -140 (region H4, SEQ ID NO: 302), -250 to -193 (region W1, SEQ ID NO: 303), -123 to -89 (region W2, SEQ ID NO: 304) and -62 to -36 (region W3, SEQ ID NO: 305) away from the transcription start site in the MITF gene promoter, and hot spot regions and warm spot regions are shown in Table 5. More specifically, one strand of the small activating nucleic acid molecule of the present invention has at least 75%, e.g., at least about 79%, about 80%, about 85%, about 90%, about 95%, about 99% or 100% homology or complementarity to any nucleotide sequence selected from SEQ ID NOs: 8-104. In a specific embodiment, one strand of the small activating nucleic acid molecule of the present invention comprises a nucleic acid sequence having at least 75%, e.g., at least about 79%, about 80%, about 85%, about 90%, about 95%, about 99% or 100% homology or complementarity to any nucleotide sequence selected from SEQ ID NOs: 8-104. In another embodiment, one strand of the small activating nucleic acid molecule of the present invention consists of a nucleic acid sequence having at least 75%, e.g., at least about 79%, about 80%, about 85%, about 90%, about 95%, about 99% or 100% homology or complementarity to any nucleotide sequence selected from SEQ ID NOs: 8-104. In yet another embodiment, one strand of the small activating nucleic acid molecule of the present invention is a nucleic acid sequence having at least 75%, e.g., at least about 79%, about 80%, about 85%, about 90%, about 95%, about 99% or 100% homology or complementarity to any nucleotide sequence selected from SEQ ID NOs: 8-104.

The small activating nucleic acid molecules of the present invention comprises a double-stranded small activating nucleic acid molecule targeting the promoter region of MITF gene, such as a small activating RNA (saRNA) molecule, which comprises a first oligonucleotide strand and a second oligonucleotide strand, wherein the first oligonucleotide strand has at least 75%, e.g., at least about 79%, about 80%, about 85%, about 90%, about 95%, about 99% or 100% homology or complementarity to a consecutive sequence of 16 to 35 nucleotides in length in positions -500 to -408 (region H1, SEQ ID NO: 299), -403 to -351 (region H2, SEQ ID NO: 300), -342 to -262 (region H3, SEQ ID NO: 301), -181 to -140 (region H4, SEQ ID NO: 302), -250 to -193 (region W1, SEQ ID NO: 303), -123 to -89 (region W2, SEQ ID NO: 304) and -62 to -36 (region W3, SEQ ID NO: 305) away from the transcription start site in the MITF gene promoter, the first oligonucleotide strand and the second oligonucleotide strand can complementarily form a double-stranded nucleic acid structure capable of activating the MITF gene expression in a cell.

The first oligonucleotide strand and the second oligonucleotide strand of the small activating nucleic acid molecule of the present invention may be present either on two different nucleic acid strands or on the same nucleic acid strand. When the first oligonucleotide strand and the second oligonucleotide strand are located on two different strands, at least one strand of the small activating nucleic acid molecule may have overhangs at 5' terminus and/or 3' terminus, e.g., overhangs of 0 to 6 nucleotides in length at 3' terminus, such as overhangs of 0, 1, 2, 3, 4, 5 or 6 nucleotides in length. Preferably, both strands of the small activating nucleic acid molecule of the present invention have overhangs; more preferably, the 3' terminus of both strands of the small activating nucleic acid molecule may have overhangs of 0 to 6 nucleotides in length, e.g., overhangs of 0, 1, 2, 3, 4, 5 or 6 nucleotides in length; and most preferably overhangs of 2 or 3 nucleotides in length. Preferably, the nucleotide of the overhang may be dT (thymine deoxynucleotide, or written as T). Preferably, the overhang at the 5' terminus and/or the 3' terminus is dTdT or dTdTdT.

The small activating nucleic acid molecule of the present invention may also comprise a small activating nucleic acid molecule capable of forming a double-stranded region hairpin structure, e.g., a single-stranded small activating RNA molecule. In one embodiment, the small activating nucleic acid molecule of the present invention comprises a single-stranded small activating RNA molecule targeting the promoter region of MITF gene, wherein the single-stranded small activating nucleic acid molecule can form a double-stranded region hairpin structure. When the first oligonucleotide strand and the second oligonucleotide strand are present on the same nucleic acid strand, preferably, the small activating nucleic acid molecule of the present invention may be a hairpin single-stranded nucleic acid molecule, wherein the first oligonucleotide strand and the second oligonucleotide strand have complementary regions capable of forming a double-stranded nucleic acid structure, and the double-stranded nucleic acid structure can promote the MITF gene expression in a cell with, for example, a RNA activation mechanism.

In the aforementioned small activating nucleic acid molecule, the first oligonucleotide strand and the second oligonucleotide strand may have 16 to 35 nucleotides (e.g., 16, 17, 18, 19, 20, 21, 22, 23, 24, 25, 26, 27, 28, 29, 30, 31, 32, 33, 34 or 35 nucleotides) in length.

In one embodiment, the first oligonucleotide strand of the small activating nucleic acid molecule of the present invention has at least 75%, e.g., at least about 79%, about 80%, about 85%, about 90%, about 95%, about 99% or 100% identity or homology or complementarity to any nucleotide sequence selected from SEQ ID NOs: 8-104, or the second oligonucleotide strand of the small activating nucleic acid molecule has at least 75%, e.g., at least about 79%, about 80%, about 85%, about 90%, about 95%, about 99% or 100% identity or homology or complementarity to any nucleotide sequence selected from SEQ ID NOs: 8-104. In one embodiment, the first oligonucleotide strand of the small activating nucleic acid molecule of the present invention comprises a nucleic acid sequence having at least 75%, e.g., at least about 79%, about 80%, about 85%, about 90%, about 95%, about 99% or 100% identity or homology or complementarity to any nucleotide sequence selected from SEQ ID NOs: 8-104, or consists of a nucleic acid sequence having at least 75%, e.g., at least about 79%, about 80%, about 85%, about 90%, about 95%, about 99% or 100% identity or homology or complementarity to any nucleotide sequence selected from SEQ ID NOs: 8-104; or the second oligonucleotide strand of the small activating nucleic acid molecule of the present invention comprises a nucleic acid sequence having at least 75%, e.g., at least about 79%, about 80%, about 85%, about 90%, about 95%, about 99% or 100% identity or homology or complementarity to any nucleotide sequence selected from SEQ ID NOs: 8-104, or consists of a nucleic acid sequence having at least 75%, e.g., at least about 79%, about 80%, about 85%, about 90%, about 95%, about 99% or 100% identity or homology or complementarity to any nucleotide sequence selected from SEQ ID NOs: 8-104. In a specific embodiment, the first oligonucleotide strand of the small activating nucleic acid molecule of the present invention may comprise any nucleotide sequence selected from SEQ ID NOs: 105-201, and/or the second oligonucleotide strand thereof may comprise any nucleotide sequence selected from SEQ ID NOs: 202-298. In one embodiment, the small activating nucleic acid molecule described herein can be synthesized, transcribed *in vitro* or expressed by a vector.

All the nucleotides in the small activating nucleic acid molecule described herein may be natural non-chemically modified nucleotides or may comprise at least one modification. In one embodiment, the modification in the small activating nucleic acid molecule described herein may comprise a chemical modification, for example, at least one nucleotide may have a chemical modification, and the chemical modification used in the present invention may comprise or be selected from one or more or any combination of the following modifications:

(1) modification of a phosphodiester bond of nucleotides in the nucleotide sequence of the small activating nucleic acid molecule;
(2) modification of 2'-OH of a ribose in the nucleotide sequence of the small activating nucleic acid molecule;
(3) modification of a base in the nucleotide of the small activating nucleic acid molecule; and
(4) at least one nucleotide in the nucleotide sequence of the small activating nucleic acid molecule being a locked nucleic acid.

The chemical modification described herein is well-known to those skilled in the art, and the modification of the phosphodiester bond refers to the modification of oxygen in the phosphodiester bond, including, but not limited to phosphorothioate modification and boranophosphate modification. Both modifications can stabilize an saRNA structure and maintain high specificity and high affinity for base pairing.

The ribose modification refers to the modification of 2'-OH in pentose of a nucleotide, i.e., the conjugation of certain substituents into hydroxyl positions of the ribose, for example, including, but not limited to 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, and the like.

The base modification refers to the modification of the base of a nucleotide, for example, including, but not limited to 5'-bromouracil modification, 5'-iodouracil modification, N-methyluracil modification, 2,6-diaminopurine modification, and the like.

These modifications can increase the bioavailability of the small activating nucleic acid molecule, improve affinity to a target sequence and enhance resistance to nuclease hydrolysis in a cell.

In addition, in order to promote the access of the small activating nucleic acid molecule into a cell, on the basis of the aforementioned modifications, a lipophilic group such as cholesterol can be conjugated into the terminus of the first oligonucleotide strand and/or the second oligonucleotide strand of the small activating nucleic acid molecule, to facilitate the interaction with the gene promoter region in the cell nucleus through the cell membrane and nuclear membrane composed of lipid bilayers.

After contacting a cell or introducing into a cell, the small activating nucleic acid molecule provided by the present invention can effectively activate or upregulate the MITF gene expression in the cell, preferably by at least 30%.

Another aspect of the present invention also relates to a nucleic acid coding the small activating nucleic acid molecule described herein. In one embodiment, the nucleic acid may be a DNA molecule. In one embodiment, the nucleic acid is an expression vector comprising a fragment encoding the small activating nucleic acid molecule described herein, which upon introduction into a cell, can express the small activating nucleic acid molecule described herein.

Another aspect of the present invention provides a cell comprising the aforementioned small activating nucleic acid molecule or the nucleic acid coding the small activating nucleic acid molecule described herein. In one embodiment, the small activating nucleic acid molecule of the present invention may be a double-stranded small activating nucleic acid molecule targeting the promoter region of MITF gene, such as a double-stranded small activating RNA (saRNA) molecule, which comprises a first oligonucleotide strand and a second oligonucleotide strand. In another embodiment, the small activating nucleic acid molecule of the present invention may be a single-stranded small activating nucleic acid molecule targeting the promoter region of MITF gene, such as a double-stranded small activating RNA (saRNA) molecule.

Another aspect of the present invention provides a composition (such as a pharmaceutical composition). The composition comprises the small activating nucleic acid molecule of the present invention or the nucleic acid coding the small activating nucleic acid molecule described herein and, optionally, a pharmaceutically acceptable carrier. In one embodiment, the pharmaceutically acceptable carrier may comprise or be selected from a liposome, a macromolecular polymer and a polypeptide.

In another aspect of the present invention, provided is a formulation, which comprises: the small activating nucleic acid molecule described herein, the nucleic acid coding the small activating nucleic acid molecule described herein, the cell comprising the small activating nucleic acid molecule of the present invention or the nucleic acid coding the small activating nucleic acid molecule of the present invention, or the composition comprising the small activating nucleic acid molecule of the present invention.

In another aspect of the present invention, provided is a kit, which comprises: the small activating nucleic acid molecule of the present invention, the nucleic acid coding the small activating nucleic acid molecule described herein, the cell comprising the small activating nucleic acid molecule of the present invention or the nucleic acid coding the small activating nucleic acid molecule of the present invention, or the composition comprising the small activating nucleic acid molecule of the present invention.

Another aspect of the present invention relates to use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention to prepare a drug or formulation for activating / upregulating the MITF gene expression in a cell.

Another aspect of the present invention also relates to a method for activating / upregulating the MITF gene expression in a cell. The method comprises administering the small activating nucleic acid molecule of the present invention, the nucleic acid coding the small activating nucleic acid molecule of the present invention, or the composition or formulation comprising the small activating nucleic acid molecule of the present invention to the cell. In one embodiment, the method for activating / upregulating the MITF gene expression in a cell comprises administering the small activating nucleic acid molecule of the present invention, the nucleic acid coding the small activating nucleic acid molecule of the present invention, or the composition comprising the small activating nucleic acid molecule of the present invention to the cell. The above cells include mammal cells, for example, cells from human, such as human epidermal melanocytes (HEMs) and normal human epidermal keratinocytes (NHEKs), which may be *in vitro* or may present in a mammal body, such as in a human body.

The small activating nucleic acid molecule of the present invention can be directly introduced into a cell, or can be produced in the cell after a nucleotide sequence coding the small activating nucleic acid molecule of the present invention is introduced into the cell. The cell is preferably a mammal cell, more preferably a human cell. The aforementioned cell may be *in vitro,* such as a cell line or a cell strain, or may present in a mammal body, such as a human body. The human body may be a patient suffering from a disease or condition related to insufficient or decreased MITF protein expression. The small activating nucleic acid molecule of the present invention can be administered at a sufficient dose to treat the disease or condition related to deficiency of the amount of MITF protein or insufficient or decreased MITF protein expression. Specifically, the disease or condition related to deficiency of the amount of MITF protein or insufficient or decreased MITF protein expression may include, for example, vitiligo.

Another aspect of the present invention provides an isolated small activating nucleic acid molecule target site of MITF gene, which has any consecutive sequence of 16 to 35 nucleotides in length in the promoter region (SEQ ID NO: 1) of MITF gene, and preferably, the target site comprises or is selected from a sequence of any 16 to 35 contiguous nucleotides of any sequence selected from SEQ ID NOs: 299-305. Specifically, the target site may comprise or be selected from any nucleotide sequence set forth in SEQ ID NOs: 8-104.

Another aspect of the present invention relates to a method for treating a disease or condition related to insufficient or decreased MITF protein expression in an individual, which comprises administering to the individual a therapeutic effective dose of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention. In one embodiment, the method for treating a disease or condition related to insufficient or decreased MITF protein expression in an individual in the present invention comprises administering to the individual a therapeutic effective dose of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention, and therapeutic effective doses of other agents, wherein the other agents comprise, for example, a micromolecular compound, an antibody, a polypeptide and a protein. The individual may be a mammal including, for example, a human. In one embodiment, the disease or condition related to insufficient or decreased MITF protein expression may comprise, for example, vitiligo. Vitiligo can be divided into vitiligo vulgaris and segmental vitiligo according to clinical features, wherein the vitiligo vulgaris is related to the melanocyte impairment caused by autoimmune regulation or oxidative stress, and the segmental vitiligo is related to genetic factors.

Vitiligo is related to factors such as familial hereditary, autoimmune regulation, oxidative stress and functional melanocyte impairment.

Another aspect of the present invention relates to a method for treating vitiligo in an individual, which comprises administering to the individual a therapeutic effective dose of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention. In one embodiment, the method for treating vitiligo in an individual in the present invention comprises administering to the individual a therapeutic effective dose of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention, and therapeutic effective doses of other agents, wherein the other agents comprise, for example, a micromolecular compound, an antibody, a polypeptide and a protein. The individual may be a mammal including, for example, a human. In the above method, the vitiligo includes vitiligo vulgaris, segmental vitiligo and the like. In the above method, the individual include a mammal, for example, a human.

Another aspect of the present invention relates to use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention to prepare a drug for treating a disease or condition related to insufficient or decreased MITF protein expression. The individual may be a mammal, for example, a human. In one embodiment, the disease related to insufficient or decreased MITF protein expression may comprise, for example, vitiligo. Preferably, the disease or condition related to insufficient or decreased MITF protein expression is vitiligo, more preferably, the disease or condition related to insufficient or decreased MITF protein expression comprises vitiligo vulgaris, segmental vitiligo and the like.

In one embodiment, provided is the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition comprising the small activating nucleic acid molecule of the present invention for use to prepare a drug for treating a disease such as vitiligo related to insufficient or decreased MITF protein expression. In one embodiment, the disease related to insufficient or decreased MITF protein expression may comprise, for example, vitiligo. Preferably, the disease or condition related to insufficient or decreased MITF protein expression is vitiligo, more preferably, the disease or condition related to insufficient or decreased MITF protein expression comprises vitiligo vulgaris, segmental vitiligo and the like.

Another aspect of the present invention relates to use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition or formulation comprising the small activating nucleic acid molecule of the present invention to prepare a drug or pharmaceutical composition for treating vitiligo. Preferably, the vitiligo includes vitiligo vulgaris, segmental vitiligo and the like.

In one embodiment, provided is use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention, or the composition or formulation comprising the small activating nucleic acid molecule of the present invention to prepare a drug or pharmaceutical composition for treating a disease related to insufficient or decreased MITF protein expression.

Compared with the prior art, the present invention has the following beneficial effects in one aspect or several aspects:

the small activating nucleic acid molecule capable of activating / upregulating the MITF gene expression such as small activating RNA (saRNA) provided by the present invention can durably activate MITF gene, so that the MITF gene and protein expression can be efficiently and specifically upregulated or restored, and meanwhile, the small activating nucleic acid molecule has low toxic and side effects, and can be used for treating a diseases or symptom related to insufficient or decreased MITF protein expression, such as vitiligo, or preparing a drug or formulation for treating a disease or symptoms related to insufficient or decreased MITF protein expression, such as Waardenburg syndrome type 2A or Tietze syndrome.

**BRIEF DESCRIPTION OF THE DRAWINGS**

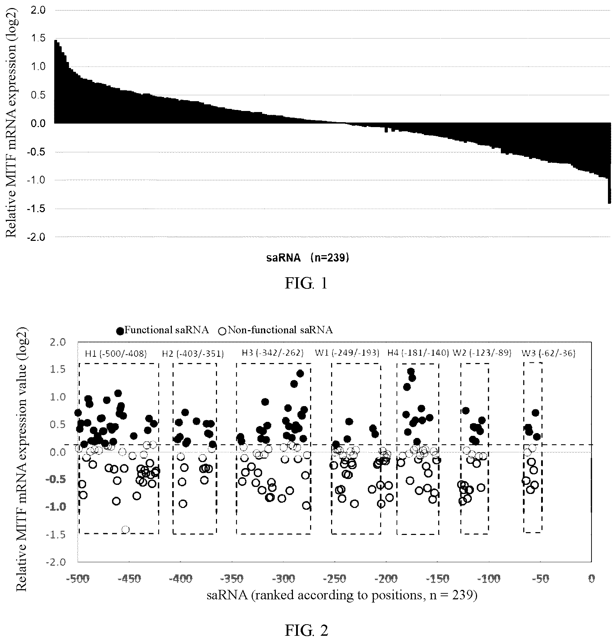

FIG. 1 shows changes in the human MITF mRNA expression mediated by saRNA. 239 saRNAs targeting the human MITF promoter were transfected into HEM cells at a final concentration of 25 nM. 72 h after the transfection, the MITF mRNA expression was analyzed by one-step RT-qPCR. Shown in the drawing is the descending rank of changes in the MITF mRNA expression (log2) relative to control treatment (Mock). The ordinate values represent the mean $\pm$ SD of 2 replicates.

FIG. 2 shows hot spot regions where saRNAs are on the human MITF promoter. 239 saRNAs targeting the human MITF promoter were transfected into HEM cells at a final concentration of 25 nM for 72 h. After the transfection was completed, the MITF mRNA expression was analyzed by one-step RT-qPCR. Shown in the drawing is changes in the MITF expression relative to control treatment (Mock) ranked from -500 to -0 according to the target positions of the saRNA in the MITF promoter. The black filled dots represent functional saRNAs, the white hollow dots represent non-functional saRNAs, and the dotted frames represent hot spot regions (H1-H4) and warm spot regions (W1-W3) of functional saRNAs. The ordinate values represent the mean $\pm$ SD of 2 replicates.

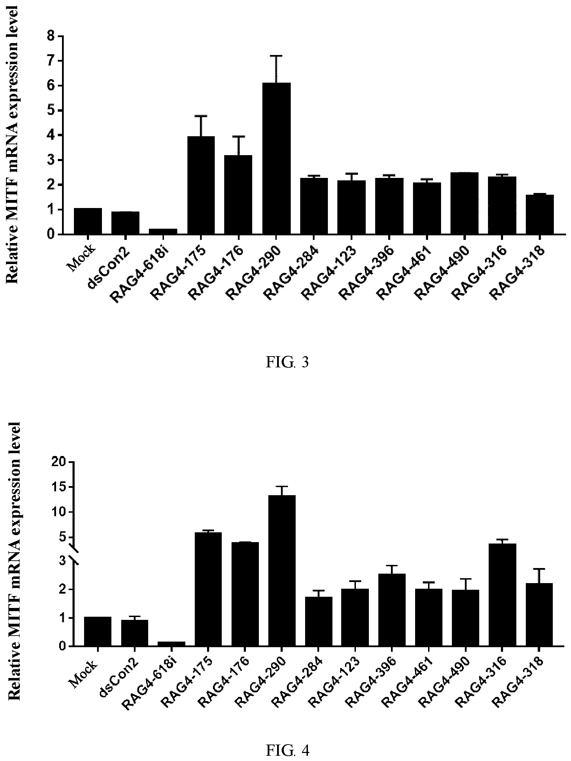

FIG. 3 shows high-throughput screening results validated by two-step RT-qPCR. saRNAs shown in FIG. 3 were transfected into HEM cells at a final concentration of 25 nM for 72 h. After transfection was completed, RNA was extracted using Qiagen RNeasy kit. After reverse transcription, an ABI 7500 FAST real-time PCR system was used to perform qPCR amplification. At the same time, HPRT1 gene was amplified as an internal reference. Shown in the drawing is relative MITF mRNA expression value after treatment of cells with a single saRNA. Mock, dsCon2 and RAG4-618i are blank transfection, non-specific control duplex RNA transfection and small interfering RNA control transfection, respectively. The ordinate values represent the mean $\pm$ SD of 2 replicates.

FIG. 4 shows high-throughput screening results validated by two-step RT-qPCR. saRNAs shown in FIG. 4 were transfected into NHEK cells at a final concentration of 25 nM for 72 h. After transfection was completed, RNA was extracted using Qiagen RNeasy kit. After reverse transcription, an ABI 7500 FAST real-time PCR system was used to perform qPCR amplification. At the same time, HPRT1 gene was amplified as an internal reference. Shown in the drawing is relative MITF mRNA expression value after treatment of cells with a single saRNA. Mock, dsCon2 and RAG4-618i are blank transfection, non-specific control duplex RNA transfection and small interfering RNA control transfection, respectively. The ordinate values represent the mean $\pm$ SD of 2 replicates.

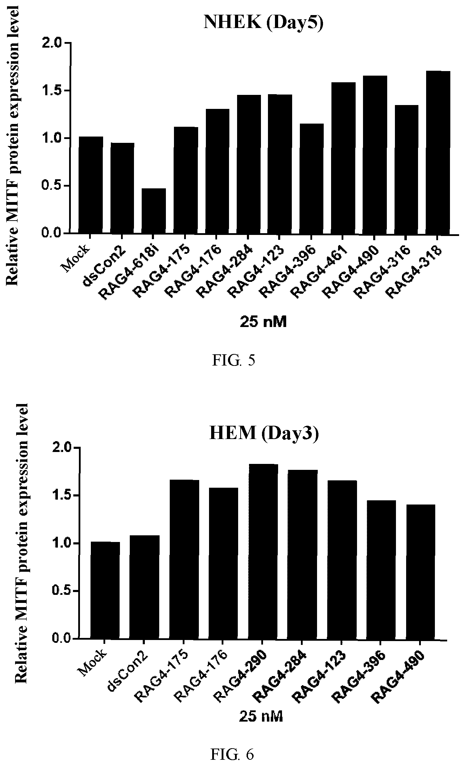

FIG. 5 shows that saRNAs promote MITF protein expression in human NHEK cells. saRNAs shown in FIG. 5 were transfected into NHEK cells at a final concentration of 25 nM for 5 days. After cells were collected and the total cell protein was extracted, the MITF protein expression level was detected using western blot. At the same time, Tubulin ($\alpha/\beta$-Tubulin) was detected as an internal control. Shown in the drawing is relative MITF protein expression value of single saRNA-treated cell. Mock, dsCon2 and RAG4-618i are blank transfection, non-specific control duplex RNA transfection and small interfering RNA control transfection, respectively.

FIG. 6 shows that saRNAs promote MITF protein expression in human HEM cells. saRNAs shown in FIG. 6 were transfected into HEK cells at a final concentration of 25 nM for 72 h. After cells were collected and the total cell protein was extracted, the MITF protein expression level was detected using western blot. At the same time, Tubulin ($\alpha/\beta$-Tubulin) was detected as an internal control. Shown in the drawing is relative MITF protein expression value of single saRNA-treated cell. Mock, dsCon2 and RAG4-618i are blank transfection, non-specific control duplex RNA transfection and small interfering RNA control transfection, respectively.

**DETAILED DESCRIPTION**

In the present invention, the related terms are defined as follows.

The term "complementarity" as used herein refers to the capability of forming base pairs between two oligonucleotide strands. The base pairs are generally formed through hydrogen bonds between nucleotides in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing).

Complementarity includes complete complementarity and incomplete complementarity. "Complete complementarity" or "100% complementarity" means that each nucleotide from the first oligonucleotide strand can form a hydrogen bond with a nucleotide at a corresponding position in the second oligonucleotide strand in the double-stranded region of the double-stranded oligonucleotide molecule without "mispairing". "Incomplete complementarity" means that not all the nucleotide units of the two strands are bonded with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity refers to at least about 75%, about 79%, about 80%, about 85%, about 90%, about 95%, about 99% or 100% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribosyl portions and ribosyl portions, as well as modified and naturally or unnaturally existing frameworks for such oligonucleotides. The oligonucleotide for activating target gene transcription described herein is a small activating nucleic acid molecule.

The terms "oligonucleotide strand" and "oligonucleotide sequence" as used herein can be used interchangeably, referring to a generic term for short nucleotide sequences having less than 35 bases (including nucleotide strands including deoxyribonucleic acid DNA or ribonucleic acid RNA, and also including mixed oligonucleotide strands formed collectively from one or more deoxyribonucleotides and one or more ribonucleotides). In the present invention, an oligonucleotide strand may have any of 16 to 35 nucleotides in length.

As used herein, the term "first oligonucleotide strand" may be a sense strand or an antisense strand. The sense strand of a small activating RNA refers to a nucleic acid strand contained in a small activating RNA duplex which has identity to the coding strand of the promoter DNA sequence of a target gene, and the antisense strand refers to a nucleic acid strand in the small activating RNA duplex which is complementary to the sense strand.

As used herein, the term "second oligonucleotide strand" can also be a sense strand or an antisense strand. If the first oligonucleotide strand is a sense strand, the second oligonucleotide strand is an antisense strand; and if the first oligonucleotide strand is an antisense strand, the second oligonucleotide strand is a sense strand.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for encoding a promoter are introduced into a host cell, or a heterogenous promoter adjacent to an endogenous coding sequence is introduced into a host cell). For example, the term "gene" comprises a nucleic acid sequence consisting of exons and introns. Protein-coding sequences are, for example, sequences contained within exons in an open reading frame between an initiation codon and a termination codon, and as used herein, "gene" can comprise such as a gene regulatory sequence, such as a promoter, an enhancer, and all other sequences known in the art for controlling the transcription, expression or activity of another gene, no matter whether the gene comprises a coding sequence or a non-coding sequence. In one case, for example, "gene" can be used to describe a functional nucleic acid comprising a regulatory sequence such as a promoter or an enhancer. The expression of a recombinant gene can be controlled by one or more types of heterogenous regulatory sequences.

The term "target gene" as used herein can refer to nucleic acid sequences naturally present in organisms, transgenes, viral or bacterial sequences, can be chromosomes or extrachromosomal genes, and/or can be transiently or stably transfected or incorporated into cells and/or chromatins thereof. The target gene can be a protein-coding gene or a non-protein-coding gene (such as a microRNA gene and a long non-coding RNA gene). The target gene generally contains a promoter sequence, and the positive regulation for the target gene can be achieved by designing a small activating nucleic acid molecule having sequence identity (also called homology) to the promoter sequence, characterized as the upregulation of expression of the target gene. "Sequence of a target gene promoter" refers to a non-coding sequence of the target gene, and the reference of the sequence of a target gene promoter in the phrase "complementary to the sequence of a target gene promoter" of the present invention refers to a coding strand of the sequence, also known as a non-template strand, i.e., a nucleic acid sequence having the same sequence as the coding sequence of the gene. "Target" or "target sequence" refers to a sequence fragment in the sequence of a target gene promoter that is homologous or complementary with a sense oligonucleotide strand or an antisense oligonucleotide strand of a small activating nucleic acid molecule.

As used herein, the terms "sense strand" and "sense nucleic acid strand" can be used interchangeably, and the sense oligonucleotide strand of a small activating nucleic acid molecule refers to the first oligonucleotide strand having identity to the coding strand of the sequence of a target gene promoter in the small activating nucleic acid molecule duplex.

As used herein, the terms "antisense strand" and "antisense nucleic acid strand" can be used interchangeably, and the antisense oligonucleotide strand of a small activating nucleic acid molecule refers to the second oligonucleotide strand complementary with the sense oligonucleotide strand in the small activating nucleic acid molecule duplex.

The term "coding strand" as used herein refers to a DNA strand in the target gene which cannot be used for transcription, and the nucleotide sequence of this strand is the same as that of an RNA produced from transcription (in the RNA, T in DNA is replaced by U). The coding strand of the double-stranded DNA sequence of the target gene promoter described herein refers to a promoter sequence on the same DNA strand as the DNA coding strand of the target gene.

The term "template strand" as used herein refers to the other strand complementary with the coding strand in the double-stranded DNA of the target gene, i.e., the strand that, as a template, can be transcribed into RNA, and this strand is complementary with the transcribed RNA (A to U and G to C). In the process of transcription, RNA polymerase binds to the template strand, moves along the 3'→5' direction of the template strand, and catalyzes the synthesis of the RNA along the 5'→3' direction. The template strand of the double-stranded DNA sequence of the target gene promoter described herein refers to a promoter sequence on the same DNA strand as the DNA template strand of the target gene.

The term "promoter" as used herein refers to a sequence which plays a regulatory role for the transcription of a protein-coding or RNA-coding nucleic acid sequence by associating with them spatially. Generally, a eukaryotic gene promoter contains 100 to 5000 base pairs, although this length range is not intended to limit the term "promoter" as used herein. Although the promoter sequence is generally located at the 5' terminus of a protein-coding or RNA-coding sequence, the promoter sequence may also exist in exon and intron sequences.

The term "transcription start site" as used herein refers to a nucleotide marking the transcription start on the template strand of a gene. The transcription start site may appear on the template strand of the promoter region. A gene can have more than one transcription start site.

The term "identity" or "homology" as used herein means that one oligonucleotide strand (a sense or an antisense strand) of a small activating RNA has similarity to a coding strand or a template strand in a region of the promoter sequence of a target gene. As used herein, the "identity" or "homology" may be at least about 75%, about 79%, about 80%, about 85%, about 90%, about 95%, about 99% or 100%.

The term "overhang" as used herein refers to non-base-paired nucleotides at the terminus (5' or 3') of an oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation", "activating gene expression", "gene upregulation" and "upregulating gene expression" can be used interchangeably, and mean an increase in transcription, translation, expression or activity of a certain nucleic acid as determined by measuring the transcriptional level, mRNA level, protein level, enzymatic activity, methylation state, chromatin state or configuration, translation level or the activity or state in a cell or biological system of a gene. These activities or states can be determined directly or indirectly. In addition, "gene activation", "activating gene expression", "gene upregulation" or "upregulating gene expression" refers to an increase in activity associated with a nucleic acid sequence, regardless of the mechanism of such activation. For example, the nucleic acid sequence plays a regulatory role as a regulatory sequence, the nucleic acid sequence is transcribed into RNA and the RNA is translated into a protein, thereby increasing the protein expression.

As used herein, the terms "small activating RNA", "saRNA", and "small activating nucleic acid molecule" can be used interchangeably, and refer to a nucleic acid molecule that can upregulate target gene expression and can be composed of the first nucleic acid fragment (antisense nucleic acid strand, also referred to as antisense oligonucleotide strand) containing a nucleotide sequence having sequence identity or homology with the non-coding nucleic acid sequence (e.g., a promoter and an enhancer) of a target gene and the second nucleic acid fragment (sense nucleic acid strand, also referred to as sense oligonucleotide strand) containing a nucleotide sequence complementary with the first nucleic acid fragment, wherein the first nucleic acid fragment and the second nucleic acid fragment form a duplex. The small activating nucleic acid molecule can also be composed of a synthesized or vector-expressed single-stranded RNA molecule that can form a hairpin structure by two complementary regions within the molecule, wherein the first region comprises a nucleotide sequence having sequence identity to the target sequence of a promoter of a gene, and the second region comprises a nucleotide sequence which is complementary to the first region. The length of the duplex region of the small activating nucleic acid molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45, or about 50 base pairs. In addition, the terms "saRNA", "small activating RNA", and "small activating nucleic acid molecule" also comprise nucleic acids other than the ribonucleotide, including, but not limited to, modified nucleotides or analogues.

As used herein, the term "hot spot (H region)" refers to a gene promoter region of at least 25 bp in length. The gathering of targets of functional small activating nucleic acid molecules appears in these hot spot regions, wherein at least 30% of the small activating nucleic acid molecules targeting these hot spot regions can induce 1.1 folds or more change in the mRNA expression of a target gene; the term "warm spot (W region)" refers to a gene promoter region of at least 25 bp in length. The gathering of targets of functional small activating nucleic acid molecules appears in these warm spot regions, wherein 8% to 30% of the small activating nucleic acid molecules targeting these warm spot regions can induce 1.1 folds or more change in the mRNA expression of a target gene.

As used herein, the term "synthesis" refers to a method for synthesis of an oligonucleotide, including any method allowing RNA synthesis, such as chemical synthesis, *in vitro* transcription, and/or vector-based expression.

According to the present invention, the MITF gene expression is upregulated by RNA activation, and a related disease (particularly vitiligo) is treated by increasing the MITF protein expression level. The MITF gene in the present invention is sometimes also called a target gene.

The method for preparing the small activating nucleic acid molecule provided by the present invention comprises sequence design and sequence synthesis.

The synthesis of the sequence of the small activating nucleic acid molecule of the present invention can be performed by adopting a chemical synthesis or can be entrusted to a biotechnology company specialized in nucleic acid synthesis.

Generally speaking, the chemical synthesis comprises the following four steps: (1) synthesis of oligomeric ribonucleotides; (2) deprotection; (3) purification and isolation; and (4) desalination and annealing.

For example, the specific steps for chemically synthesizing the saRNA described herein are as follows.

(1) Synthesis of oligomeric ribonucleotides

Synthesis of 1 $\mu$M RNA was set in an automatic DNA/RNA synthesizer (e.g., Applied Biosystems EXPEDITE8909), and the coupling time of each cycle was also set as 10 to 15 min. With a solid phase-bonded 5'-O-p-dimethoxytri phenylmethyl -thymidine substrate as an initiator, one base was bonded to the solid phase substrate in the first cycle, and then, in the $n^{th}$ ($19 \geq n \geq 2$) cycle, one base was bonded to the base bonded in the $n-1^{th}$ cycle. This process was repeated until the synthesis of the whole nucleic acid sequence was completed.

(2) Deprotection

The solid phase substrate bonded with the saRNA was put into a test tube, and 1 mL of a mixed solution of ethanol and ammonium hydroxide (volume ratio: 1:3) was added to the test tube. The test tube was then sealed and placed in an incubator, and the mixture was incubated at 25-70 °C for 2 to 30 h. The solution containing the solid phase substrate bonded with the saRNA was filtered, and the filtrate was collected. The solid phase substrate was rinsed with double distilled water twice (1 mL each time), and the filtrate was collected. The collected eluent was combined and dried under vacuum for 1 to 12 h. Then the solution was added with 1 mL of a solution of tetrabutylammonium fluoride in tetrahydrofuran (1 M), let stand at room temperature for 4 to 12 h, followed by addition of 2 mL of n-butanol. Precipitate was collected to give a single-stranded crude product of saRNA by high-speed centrifugation.

(3) Purification and isolation

The resulting crude product of saRNA was dissolved in 2 mL of triethylamine acetate solution with a concentration of 1 mol/L, and the solution was separated by a reversed-phase C18 column of high pressure liquid chromatography to give a purified single-stranded product of saRNA.

(4) Desalination and annealing

Salts were removed by gel filtration (size exclusion chromatography). A single sense oligomeric ribonucleic acid strand and a single antisense oligomeric ribonucleic acid strand were mixed in a 1 to 2 mL of buffer (10 mM Tris, pH = 7.5-8.0, 50 mM NaCl) at a molar ratio of 1:1. The solution was heated to 95 °C, and was then slowly cooled to room temperature to give a solution containing saRNA.

It was discovered in this study that after being introduced into a cell, the aforementioned saRNA could effectively increase the MITF mRNA and protein expression.

The present invention will be further illustrated with reference to specific examples and drawings below. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: Laboratory Manual (New York: Cold Spring Harbor Laboratory Press, 1989), or conditions recommended by the manufacturer.

Embodiments of the present disclosure will be described in detail below with reference to examples, but those skilled in the art will appreciate that the following examples are only illustrative of the present disclosure and should not be construed as limiting the scope of the present disclosure. If specific conditions are not specified in the examples, conventional conditions or conditions recommended by the manufacturers shall be adopted. Reagents or instruments without specified manufacturers used herein are conventional products that are commercially available.

**Example 1. Design and Synthesis of saRNA Targeting Human MITF Promoter**

To screen a functional small activating RNA capable of activating the MITF gene expression, with a MITF promoter sequence of 500 bp in length as a template, a target with a length of 19 bp was selected from -500 bp upstream of TSS. The target sequences were then filtered to keep those which met the following criteria: (1) GC content between 40% and 65%; (2) with less than 5 consecutive identical nucleotides; (3) with 3 or less dinucleotide repeat sequences; (4) with 3 or less trinucleotide repeat sequences. After the filtration, the remaining 239 target sequences entered the screening process as candidates. Corresponding double-stranded small activating RNAs were chemically synthesized based on these candidate sequences.

Each of the sense strand and antisense strand in the double-stranded small activating RNA used in the study had 21 nucleotides in length. The 19 nucleotides in the 5' region of the first ribonucleic acid strand (sense strand) of the double-stranded saRNA had 100% identity with the target sequence of the promoter, and the 3' terminus of the first ribonucleic acid strand contained a TT sequence. The 19 nucleotides in the 5' region of the second ribonucleic acid strand were complementary with the first ribonucleic acid strand sequence, and the 3' terminus of the second ribonucleic acid strand contained a TT sequence. The aforementioned two strands of the double-stranded saRNA were mixed at a molar ratio of 1:1. After annealing, a double-stranded saRNA was formed.

The sequence of the human MITF promoter is shown as follows, which corresponds to position 1 to position 500 from 5' to 3' of SEQ ID NO: 1 in the sequence listing:

```
-500  gcgaaggaaa  gttcttcctc  gttgttccaa  tccgaggaca  agctgatatg

-450  tcgcagcagc  ccagggaagc  atgcgagctg  ataggaagtc  cttttatttt

-400  aagacaggct  cgaatgctaa  aactttcttg  tgccaaaacc  cttgactatt

-350  ttatttttaa  aataagcact  tggcgtgccc  tcgcagatgt  ctgagctgag

-300  aggtcggggc  gatggtagaa  gagcagtcag  tgtccattct  tattcatatt

-250  aagtagccaa  gtctgtaccc  ttgaagcaag  tggggagaga  ggagggagag

-200  gagctgctga  cattgacaat  gaatccaaac  aggagttgca  ctagcggtgt

-150  ccaccacgtt  gcctctcccc  cgcctggcct  tctgggagct  gtagttttcg

-100  tgggagcggc  tccccaggcg  agctgggaat  gccccgcccg  ggccgaacta

-50   cagatcccag  gcggcgctcg  gccgccagcc  cctcccgccc  gggtgcgagt
```

**Example 2. High-throughput Screening of saRNAs Targeting Human MITF Promoter**

(1) Cell culture and transfection

Human epidermal melanocytes (HEMs) (purchased from Beina Chuanglian Biotechnology Co., Ltd (Beijing), BNCC350795) and normal human epidermal keratinocytes (NHEKs) (purchased from Beina Chuanglian Biotechnology Co., Ltd (Beijing), BNCC340593) were cultured in DMEM media (Gibco) containing 10% newborn calf serum (Sigma-Aldrich) and 1% penicillin/streptomycin (Gibco). The cells were cultured at 5% $CO_2$ and 37 °C. The HEM cells were plated at 2000 cells/well into a 96-well plate; according to the instructions provided by the manufacturer, RNAiMax (Invitrogen, Carlsbad, CA) was used to transfect small activating RNAs at a concentration of 25 nM (unless otherwise specified) for 72 h, and 2 replicate wells were used for each treatment.

(2) One-step RT-qPCR

After transfection, the media were discarded, and each well was washed with 150 μL of PBS once. After PBS was discarded, each well was added with 50 μL of cell lysis solution, and incubation was performed at room temperature for 5 min. 1 μL of cell lysis solution was taken from each well and subjected to qPCR analysis on an ABI 7500 fast real-time PCR system (Applied Biosystems) using a one-step TB GreenTM PrimeScripTM RT-PCR kit II (Takara, RR086A), and each sample was repeatedly amplified in 3 replicate wells. PCR reaction conditions are shown in Table 1 below.

Table 1. PCR reaction preparation

| Reagent | Volume |
|---|---|
| 2 × One-step TB Green RT-PCR buffer 4 | 2.5 μl |
| Prime Script 1 step enzyme Mix 2 | 0.2 μl |
| Forward and reverse primers Mix (5 μM) | 0.4 μl |
| No RNase dH$_2$O | 1.4 μl |
| Crude lysate (RNA) | 0.5 μl |
| Sum | 5 μl |

Reaction conditions were as follows: reverse transcription reaction (stage 1): 5 min at 42 °C, 10 s at 95 °C; PCR reaction (stage 2): 5 s at 95 °C, 20 s at 60 °C, 45 cycles of amplification. HPRT1 and TBP were used as internal reference genes. PCR primers used by MITF, HPRT1 and TBP are shown in Table 2, wherein MITF was amplified using the MITF F1/R1 primer pair.

Table 2. Primer sequences for RT-qPCR analysis

| Primer | SEQ ID NO | Sequence (5'-3') |
|---|---|---|
| MITF F1 | SEQ ID NO:2 | CGACAGAAGAAACTGGAGGCAC |
| MITF R1 | SEQ ID NO:3 | CCCGTGGATGGAATAAGGGAAA |
| HPRT1 F | SEQ ID NO:4 | AAAGATGGTCAAGGTCGCAAG |
| HPRT1 R | SEQ ID NO:5 | TAGTCAAGGGCATATCCTACAAC |
| TBP F | SEQ ID NO:6 | TGCTCACCCACCAACAATTTAG |
| TBP R | SEQ ID NO:7 | TCTGCTCTGACTTTAGCACCTG |

To calculate the expression value (Erel) of MITF (target gene) of a saRNA-transfected sample relative to control treatment (Mock), the Ct values of the target gene and the two internal reference genes were substituted into formula 1 for calculation.

$$E_{rel} = 2^{(CtTm-CtTs)} / ((2^{(CtR1m-CtR1s)} * 2^{(CtR2m-CtR2s)})^{(1/2)})$$ (formula 1)

wherein CtTm was the Ct value of the target gene from the control treatment (Mock) sample; CtTs was the Ct value of the target gene from the saRNA-treated sample; CtRIm was the Ct value of the internal reference gene 1 from the Mock-treated sample; CtR1s was the Ct value of the internal reference gene 1 from the saRNA-treated sample; CtR2m was the Ct value of the internal reference gene 2 from the control treatment sample; and CtR2s was the Ct value of the internal reference gene 2 from the saRNA-treated sample.

(3) Screening of functional saRNAs

To obtain saRNAs capable of activating MITF transcription, the aforementioned 239 saRNAs were individually transfected into HEM cells at a transfection concentration of 25 nM. 72 h after the transfection, according to the same method as described above, the cells were lysed and subjected to one-step RT-qPCR analysis to obtain the relative (compared to the control treatment group) MITF gene expression value of each saRNA-treated sample. As shown in Table 3, 29 (12.1%) saRNAs showed high activation (≥ 1.5 fold), 68 (28.5%) saRNAs showed mild activation (≥ 1.1 fold), and 142 (59.4%) saRNAs had no upregulating effect on MITF expression. The maximum activation was 2.75 fold, and the maximum inhibition was 0.38 fold, and the saRNAs with activation activity were referred to as functional saRNAs.

Table 3. High-throughput screening results of human MITF saRNA

| saRNA activity | $\log_2$ value of changes in MITF (fold) | Number of saRNAs | Percentage |
|---|---|---|---|
| High activation | $\geq$ 0.58 (1.50) ~ $\leq$1.46(2.75) | 29 | 12.1% |
| Mild activation | $\geq$ 0.13 (1.10)~ < 0.58 (1.50) | 68 | 28.5% |
| No activation effect | < 0.13 (1.10) | 142 | 59.4% |
| Sum | | 239 | 100% |

As shown in FIG. 1, it is further shown that the descending order of changes in MITF expression of human MITF saRNA. The MITF active saRNA sequence (functional saRNA sequence), the active target sequence and changes in MITF mRNA expression are shown in Table 4 (each active target sequence listed in Table 4 corresponds to SEQ ID NOs: 8-104 in the sequence listing, each saRNA sense sequence corresponds to SEQ ID NOs: 105-201 in the sequence listing, and each saRNA antisense sequence corresponds to SEQ ID NOs: 202-298 in the sequence listing). Table 5 shows different hot spot regions and warm spot regions and related sequences where functional saRNAs are located (SEQ ID NOs: 299-305).

Table 4. Functional saRNA sequences, active target sequences thereof and changes in MITF mRNA expression caused thereby

|  | saRNA | Active target sequence (5'-3') | Functional saRNA sequence | (5'-3') | Fold of changes in relative MITF mRNA expression |
|---|---|---|---|---|---|
| Hot spot region | RAG4-500 | GCGAAGGAAAGTT CTTCCT | GCGAAGGAAAGUUCUUCCUTT AGGAAGAACUUUCCUUCGCTT | (sense) (antisense) | 1.64 |
|  | RAG4-498 | GAAGGAAAGTTCT TCCTCG | GAAGGAAAGUUCUUCCUCGTT CGAGGAAGAACUUUCCUUCTT | (sense) (antisense) | 1.34 |
|  | RAG4-497 | AAGGAAAGTTCTT CCTCGT | AAGGAAAGUUCUUCCUCGUTT ACGAGGAAGAACUUUCCUUTT | (sense) (antisense) | 1.44 |
|  | RAG4-494 | GAAAGTTCTTCCT CGTTGT | GAAAGUUCUUCCUCGUUGUTT ACAACGAGGAAGAACUUUCTT | (sense) (antisense) | 1.10 |
|  | RAG4-491 | AGTTCTTCCTCGTT GTTCC | AGUUCUUCCUCGUUGUUCCTT GGAACAACGAGGAAGAACUTT | (sense) (antisense) | 1.45 |
|  | RAG4-490 | GTTCTTCCTCGTTG | GUUCUUCCUCGUUGUUCCATT | (sense) | 1.97 |
|  |  | TTCCA | UGGAACAACGAGGAAGAACTT | (antisense) |  |
|  | RAG4-489 | TTCTTCCTCGTTGT TCCAA | UUCUUCCUCGUUGUUCCAATT UUGGAACAACGAGGAAGAATT | (sense) (antisense) | 1.84 |
|  | RAG4-487 | CTTCCTCGTTGTTC CAATC | CUUCCUCGUUGUUCCAAUCTT GAUUGGAACAACGAGGAAGTT | (sense) (antisense) | 1.15 |
|  | RAG4-485 | TCCTCGTTGTTCCA ATCCG | UCCUCGUUGUUCCAAUCCGTT CGGAUUGGAACAACGAGGATT | (sense) (antisense) | 1.32 |
|  | RAG4-483 | CTCGTTGTTCCAAT CCGAG | CUCGUUGUUCCAAUCCGAGTT CUCGGAUUGGAACAACGAGTT | (sense) (antisense) | 1.16 |
|  | RAG4-482 | TCGTTGTTCCAATC CGAGG | UCGUUGUUCCAAUCCGAGGTT CCUCGGAUUGGAACAACGATT | (sense) (antisense) | 1.16 |
|  | RAG4-481 | CGTTGTTCCAATCC GAGGA | CGUUGUUCCAAUCCGAGGATT UCCUCGGAUUGGAACAACGTT | (sense) (antisense) | 1.23 |
|  | RAG4-479 | TTGTTCCAATCCGA GGACA | UUGUUCCAAUCCGAGGACATT UGUCCUCGGAUUGGAACAATT | (sense) (antisense) | 1.16 |

| | saRNA | Active target sequence (5'-3') | Functional saRNA sequence | (5'-3') | Fold of changes in relative MITF mRNA expression |
|---|---|---|---|---|---|
| | RAG4-478 | TGTTCCAATCCGA GGACAA | UGUUCCAAUCCGAGGACAATT UUGUCCUCGGAUUGGAACATT | (sense) (antisense) | 1.53 |
| | RAG4-477 | GTTCCAATCCGAG GACAAG | GUUCCAAUCCGAGGACAAGTT CUUGUCCUCGGAUUGGAACTT | (sense) (antisense) | 1.54 |
| | RAG4-476 | TTCCAATCCGAGG ACAAGC | UUCCAAUCCGAGGACAAGCTT GCUUGUCCUCGGAUUGGAATT | (sense) (antisense) | 1.31 |
| | RAG4-475 | TCCAATCCGAGGA CAAGCT | UCCAAUCCGAGGACAAGCUTT AGCUUGUCCUCGGAUUGGATT | (sense) (antisense) | 1.30 |
| | RAG4-474 | CCAATCCGAGGAC AAGCTG | CCAAUCCGAGGACAAGCUGTT CAGCUUGUCCUCGGAUUGGTT | (sense) (antisense) | 1.12 |
| | RAG4-473 | CAATCCGAGGACA AGCTGA | CAAUCCGAGGACAAGCUGATT UCAGCUUGUCCUCGGAUUGTT | (sense) (antisense) | 1.10 |
| | RAG4-472 | AATCCGAGGACAA GCTGAT | AAUCCGAGGACAAGCUGAUTT AUCAGCUUGUCCUCGGAUUTT | (sense) (antisense) | 1.93 |
| | RAG4-469 | CCGAGGACAAGCT GATATG | CCGAGGACAAGCUGAUAUGTT CAUAUCAGCUUGUCCUCGGTT | (sense) (antisense) | 1.39 |
| | RAG4-467 | GAGGACAAGCTGA TATGTC | GAGGACAAGCUGAUAUGUCTT GACAUAUCAGCUUGUCCUCTT | (sense) (antisense) | 1.33 |
| | RAG4-466 | AGGACAAGCTGAT ATGTCG | AGGACAAGCUGAUAUGUCGTT CGACAUAUCAGCUUGUCCUTT | (sense) (antisense) | 1.14 |
| | RAG4-464 | GACAAGCTGATAT GTCGCA | GACAAGCUGAUAUGUCGCATT UGCGACAUAUCAGCUUGUCTT | (sense) (antisense) | 1.37 |
| | RAG4-461 | AAGCTGATATGTCG CAGCA | AAGCUGAUAUGUCGCAGCATT UGCUGCGACAUAUCAGCUUTT | (sense) (antisense) | 210 |
| | RAG4-460 | AGCTGATATGTCGC AGCAG | AGCUGAUAUGUCGCAGCAGTT CUGCUGCGACAUAUCAGCUTT | (sense) (antisense) | 1.63 |

(continued)

| saRNA | Active target sequence (5'-3') | Functional saRNA sequence | (5'-3') | Fold of changes in relative MITF mRNA expression |
|---|---|---|---|---|
| RAG4-459 | GCTGATATGTCGCA GCAGC | GCUGAUAUGUCGCAGCAGCUU | (sense) | 1.72 |
|  |  | GCUGCUGCGACAUAUCAGCUU | (antisense) |  |
| RAG4-458 | CTGATATGTCGCAG CAGCC | CUGAUAUGUCGCAGCAGCCUU | (sense) | 1.79 |
|  |  | GGCUGCUGCGACAUAUCAGUU | (antisense) |  |
| RAG4-456 | GATATGTCGCAGCA GCCCA | GAUAUGUCGCAGCCCAUU | (sense) | 1.58 |
|  |  | UGGGCUGCUGCGACAUAUCUU | (antisense) |  |
| RAG4-446 | AGCAGCCCAGGGA | AGCAGCCCAGGGAAGCAUGUU | (sense) | 1.23 |
|  | AGCATG | CAUGCUUCCCUGGGCUGCUGCUU | (antisense) |  |
| RAG4-432 | GCATGCGAGCTGAT AGGAA | GCAUGCGAGCUGAUAGGAAUU | (sense) | 1.32 |
|  |  | UUCCUAUCAGCUCGCAUGCUU | (antisense) |  |
| RAG4-431 | CATGCGAGCTGATA GGAAG | CAUGCGAGCUGAUAGGAAGUU | (sense) | 1.53 |
|  |  | CUUCCUAUCAGCUCGCAUGUU | (antisense) |  |
| RAG4-427 | CGAGCTGATAGGA AGTCCT | CGAGCUGAUAGGAAGUCCUUU | (sense) | 1.10 |
|  |  | AGGACAUUCCUAUCAGCUCGUU | (antisense) |  |
| RAG4-426 | GAGCTGATAGGAA GTCCTT | GAGCUGAUAGGAAGUCCUUUU | (sense) | 1.43 |
|  |  | AAGGACAUUCCUAUCAGCUCUU | (antisense) |  |
| RAG4-403 | TTTAAGACAGGCT CGAATG | UUUAAGACAGGCUCGAAUGUU | (sense) | 1.17 |
|  |  | CAUUCGAGCCUGUCUUAAAUU | (antisense) |  |
| RAG4-402 | TTAAGACAGGCTC GAATGC | UUAAGACAGGCUCGAAUGCUU | (sense) | 1.46 |
|  |  | GCAUUCGAGCCUGUCUUAAUU | (antisense) |  |
| RAG4-401 | TAAGACAGGCTCG AATGCT | UAAGACAGGCUCGAAUGCUUU | (sense) | 1.21 |
|  |  | AGCAUUCGAGCCUGUCUUAUU | (antisense) |  |
| RAG4-396 | CAGGCTCGAATGC TAAAAC | CAGGCUCGAAUGCUAAAACUU | (sense) | 1.65 |
|  |  | GUUUUAGCAUUCGAGCCUGUU | (antisense) |  |
| RAG4-395 | AGGCTCGAATGCT AAAACT | AGGCUCGAAUGCUAAAACUUU | (sense) | 1.12 |
|  |  | AGUUUUAGCAUUCGAGCCUUU | (antisense) |  |

EP 4 166 668 A1

| | saRNA | Active target sequence (5'-3') | Functional saRNA sequence | (5'-3') | Fold of changes in relative MITF mRNA expression |
|---|---|---|---|---|---|
| | RAG4-394 | GGCTCGAATGCTAAAACTT | GGCUCGAAUGCUAAAACUUTT<br>AAGUUUUAGCAUUCGAGCCTT | (sense)<br>(antisense) | 1.15 |
| | RAG4-385 | GCTAAAACTTTCTTGTGCC | GCUAAAACUUUCUUGUGCCTT<br>GGCACAAGAAAGUUUUAGCTT | (sense)<br>(antisense) | 1.48 |
| | RAG4-375 | TCTTGTGCCAAAACCCTTG | UCUUGUGCCAAAACCCUUGTT<br>CAAGGGUUUUGGCACAAGATT | (sense)<br>(antisense) | 1.43 |
| | RAG4-374 | CTTGTGCCAAAACCCTTGA | CUUGUGCCAAAACCCUUGATT<br>UCAAGGGUUUUGGCACAAGTT | (sense)<br>(antisense) | 1.27 |
| | RAG4-372 | TGTGCCAAAACCCTTGACT | UGUGCCAAAACCCUUGACUTT<br>AGUCAAGGGUUUUGGCACATT | (sense)<br>(antisense) | 1.26 |
| | RAG4-371 | GTGCCAAAACCCTTGACTA | GUGCCAAAACCCUUGACUATT<br>UAGUCAAGGGUUUUGGCACTT | (sense)<br>(antisense) | 1.43 |
| | RAG4-369 | GCCAAAACCCTTGACTATT | GCCAAAACCCUUGACUAUUTT<br>AAUAGUCAAGGGUUUUGGCTT | (sense)<br>(antisense) | 1.11 |
| | RAG4-342 | AAAATAAGCACTTGGCGTG | AAAAUAAGCACUUGGCGUGTT<br>CACGCCAAGUGCUUAUUUUTT | (sense)<br>(antisense) | 1.21 |
| | RAG4-341 | AAATAAGCACTTGGCGTGC | AAAUAAGCACUUGGCGUGCTT<br>GCACGCCAAGUGCUUAUUUTT | (sense)<br>(antisense) | 1.15 |
| | RAG4-323 | CCCTCGCAGATGTCTGAGC | CCCUCGCAGAUGUCUGAGCTT<br>GCUCAGACAUCUGCGAGGGTT | (sense)<br>(antisense) | 1.32 |
| | RAG4-322 | CCTCGCAGATGTCTGAGCT | CCUCGCAGAUGUCUGAGCUTT<br>AGCUCAGACAUCUGCGAGGTT | (sense)<br>(antisense) | 1.19 |
| | RAG4-320 | TCGCAGATGTCTGAGCTGA | UCGCAGAUGUCUGAGCUGATT<br>UCAGCUCAGACAUCUGCGATT | (sense)<br>(antisense) | 1.30 |
| | RAG4-318 | GCAGATGTCTGAGCTGAGA | GCAGAUGUCUGAGCUGAGATT<br>UCUCAGCUCAGACAUCUGCTT | (sense)<br>(antisense) | 1.89 |

(continued)

| | saRNA | Active target sequence (5'-3') | Functional saRNA sequence | (5'-3') | Fold of changes in relative MITF mRNA expression |
|---|---|---|---|---|---|
| | RAG4-317 | CAGATGTCTGAGC TGAGAG | CAGAUGUCUGAGCUGAGAGTT CUCUCAGCUCAGACAUCUGTT | (sense) (antisense) | 1.17 |
| | RAG4-316 | AGATGTCTGAGCT GAGAGG | AGAUGUCUGAGCUGAGAGGTT CCUCUCAGCUCAGACAUCUTT | (sense) (antisense) | 1.40 |
| | RAG4-298 | GTCGGGGCGATGG TAGAAG | GUCGGGGCGAUGGUAGAAGTT CUUCUACCAUCGCCCCGACTT | (sense) (antisense) | 1.49 |
| | RAG4-297 | TCGGGGCGATGGT AGAAGA | UCGGGGCGAUGGUAGAAGATT UCUUCUACCAUCGCCCCGATT | (sense) (antisense) | 1.24 |
| | RAG4-296 | CGGGGCGATGGTA GAAGAG | CGGGGCGAUGGUAGAAGAGTT CUCUUCUACCAUCGCCCCGTT | (sense) (antisense) | 1.75 |
| | RAG4-295 | GGGGCGATGGTAG AAGAGC | GGGGCGAUGGUAGAAGAGCTT GCUCUUCUACCAUCGCCCCTT | (sense) (antisense) | 1.39 |
| | RAG4-293 | GGCGATGGTAGAA GAGCAG | GGCGAUGGUAGAAGAGCAGTT CUGCUCUUCUACCAUCGCCTT | (sense) (antisense) | 1.10 |
| | RAG4-292 | GCGATGGTAGAAG AGCAGT | GCGAUGGUAGAAGAGCAGUTT ACUGCUCUUCUACCAUCGCTT | (sense) (antisense) | 1.20 |
| | RAG4-290 | GATGGTAGAAGAG CAGTCA | GAUGGUAGAAGAGCAGUCATT UGACUGCUCUUCUACCAUCTT | (sense) (antisense) | 2.37 |
| | RAG4-289 | ATGGTAGAAGAGC AGTCAG | AUGGUAGAAGAGCAGUCAGTT CUGACUGCUCUUCUACCAUTT | (sense) (antisense) | 1.33 |
| | RAG4-288 | TGGTAGAAGAGCA GTCAGT | UGGUAGAAGAGCAGUCAGUTT ACUGACUGCUCUUCUACCATT | (sense) (antisense) | 1.41 |
| | RAG4-285 | TAGAAGAGCAGTC AGTGTC | UAGAAGAGCAGUCAGUGUCTT GACACUGACUGCUCUUCUATT | (sense) (antisense) | 1.35 |
| | RAG4-284 | AGAAGAGCAGTCA GTGTCC | AGAAGAGCAGUCAGUGUCCTT GGACACUGACUGCUCUUCUTT | (sense) (antisense) | 2.68 |

EP 4 166 668 A1

| saRNA | Active target sequence (5'-3') | Functional saRNA sequence | (5'-3') | Fold of changes in relative MITF mRNA expression |
|---|---|---|---|---|
| RAG4-283 | GAAGAGCAGTCAG TGTCCA | GAAGAGCAGUCAGUGUCCAUU UGGACACUGACUGCUCUUCUU | (sense) (antisense) | 1.61 |
| RAG4-282 | AAGAGCAGTCAGT GTCCAT | AAGAGCAGUCAGUGUCCAUU AUGGACACUGACUGCUCUUUU | (sense) (antisense) | 1.58 |
| RAG4-281 | AGAGCAGTCAGTG TCCATT | AGAGCAGUCAGUGUCCAUUU AAUGGACACUGACUGCUCUUU | (sense) (antisense) | 1.19 |
| RAG4-280 | GAGCAGTCAGTGT CCATTC | GAGCAGUCAGUGUCCAUUCUU GAAUGGACACUGACUGCUCUU | (sense) (antisense) | 1.71 |
| RAG4-181 | TGAATCCAAACAG GAGTTG | UGAAUCCAAACAGGAGUUGUU CAACUCCUGUUUGGAUUCAUU | (sense) (antisense) | 1.61 |
| RAG4-180 | GAATCCAAACAGG AGTTGC | GAAUCCAAACAGGAGUUGCUU GCAACUCCUGUUUGGAUUCUU | (sense) (antisense) | 2.28 |
| RAG4-179 | AATCCAAACAGGA GTTGCA | AAUCCAAACAGGAGUUGCAUU UGCAACUCCUGUUUGGAUUUU | (sense) (antisense) | 1.29 |
| RAG4-176 | CCAAACAGGAGTT GCACTA | CCAAACAGGAGUUGCACUAUU UAGUGCAACUCCUGUUUGGUU | (sense) (antisense) | 2.75 |
| RAG4-175 | CAAACAGGAGTTG CACTAG | CAAACAGGAGUUGCACUAGUU CUAGUGCAACUCCUGUUUGUU | (sense) (antisense) | 2.54 |
| RAG4-174 | AAACAGGAGTTGC ACTAGC | AAACAGGAGUUGCACUAGCUU GCUAGUGCAACUCCUGUUUUU | (sense) (antisense) | 1.44 |
| RAG4-172 | ACAGGAGTTGCAC TAGCGG | ACAGGAGUUGCACUAGCGGUU CCGCUAGUGCAACUCCUGUUU | (sense) (antisense) | 1.49 |
| RAG4-170 | AGGAGTTGCACTA GCGGTG | AGGAGUUGCACUAGCGGUGUU CACCGCUAGUGCAACUCCUUU | (sense) (antisense) | 1.14 |
| RAG4-166 | GTTGCACTAGCGG | GUUGCACUAGCGGUGUCCAUU | (sense) | 1.73 |

| | saRNA | Active target sequence (5'-3') | Functional saRNA sequence | (5'-3') | Fold of changes in relative MITF mRNA expression |
|---|---|---|---|---|---|
| | | TGTCCA | UGGACACCGCUAGUGCAACUU | (antisense) | |
| | RAG4-165 | TTGCACTAGCGGT GTCCAC | UUGCACUAGCGGUGUCCACUU GUGGACACCGCUAGUGCAAUU | (sense) (antisense) | 1.50 |
| | RAG4-158 | AGCGGTGTCCACC ACGTTG | AGCGGUGUCCACCACGUUGUU CAACGUGGUGGACACCGCUUU | (sense) (antisense) | 1.55 |
| Warm spot region | RAG4-249 | AGTAGCCAAGTCT GTACCC | AGUAGCCAAGUCUGUACCCUU GGGUACAGACUUGGCUACUUU | (sense) (antisense) | 1.10 |
| | RAG4-238 | CTGTACCCTTGAA GCAAGT | CUGUACCCUUGAAGCAAGUUU ACUUGCUUCAAGGGUACAGUU | (sense) (antisense) | 1.18 |
| | RAG4-236 | GTACCCTTGAAGC AAGTGG | GUACCCUUGAAGCAAGUGGUU CCACUUGCUUCAAGGGUACUU | (sense) (antisense) | 1.47 |
| | RAG4-213 | AGAGGAGGGAGA GGAGCTG | AGAGGAGGGAGAGGAGCUGUU CAGCUCCUCUCCCUCCUCUUU | (sense) (antisense) | 1.35 |
| | RAG4-211 | AGGAGGGAGAGG AGCTGCT | AGGAGGGAGAGGAGCUGCUUU AGCAGCUCCUCUCCCUCCUUU | (sense) (antisense) | 1.25 |
| | RAG4-123 | CCTTCTGGGAGCT GTAGTT | CCUUCUGGGAGCUGUAGUUUU AACUACAGCUCCCAGAAGGUU | (sense) (antisense) | 1.69 |
| | RAG4-116 | GGAGCTGTAGTTTT CGTGG | GGAGCUGUAGUUUUCGUGGUU CCACGAAAACUACAGCUCCUU | (sense) (antisense) | 1.17 |
| | RAG4-115 | GAGCTGTAGTTTTC GTGGG | GAGCUGUAGUUUUCGUGGGUU CCCACGAAAACUACAGCUCUU | (sense) (antisense) | 1.38 |
| | RAG4-114 | AGCTGTAGTTTTCG TGGGA | AGCUGUAGUUUUCGUGGGAUU UCCCACGAAAACUACAGCUUU | (sense) (antisense) | 1.36 |
| | RAG4-113 | GCTGTAGTTTTCGT GGGAG | GCUGUAGUUUUCGUGGGAGUU CUCCCACGAAAACUACAGCUU | (sense) (antisense) | 1.14 |
| | RAG4-111 | TGTAGTTTTCGTGG GAGCG | UGUAGUUUUCGUGGGAGCGUU CGCUCCCACGAAAACUACAUU | (sense) (antisense) | 1.36 |

21

(continued)

| saRNA | Active target sequence (5'-3') | Functional saRNA sequence | (5'-3') | Fold of changes in relative MITF mRNA expression |
|---|---|---|---|---|
| RAG4-109 | TAGTTTTCGTGGGA GCGGC | UAGUUUUCGUGGGAGCGGCUU (sense)<br>GCCGCUCCCACGAAAACUAUU (antisense) | | 1.30 |
| RAG4-107 | GTTTTCGTGTGGGAG CGGCTC | GUUUUCGUGGGAGCGGCUCUU (sense)<br>GAGCCGCUCCCACGAAAACUU (antisense) | | 1.49 |
| RAG4-62 | CGGGGCCGAACTAC AGATCC | CGGGCCGAACUACAGAUCCUU (sense)<br>GGAUCUGUAGUUCGGCCCGUU (antisense) | | 1.36 |
| RAG4-61 | GGGGCCGAACTACA GATCCC | GGGCCGAACUACAGAUCCCUU (sense)<br>GGGAUCUGUAGUUCGGCCCUU (antisense) | | 1.29 |
| RAG4-55 | AACTACAGATCCC AGGCGG | AACUACAGAUCCCAGGCGGUU (sense)<br>CCGCCUGGGAUCUGUAGUUUU (antisense) | | 1.64 |
| RAG4-54 | ACTACAGATCCCA GGCGGC | ACUACAGAUCCCAGGCGGCUU (sense)<br>GCCGCCUGGGAUCUGUAGUUU (antisense) | | 1.21 |

Table 5. Hot spot and warm spot regions and sequences of functional saRNAs

| Hot spot region | Sequence of hot spot region |
|---|---|
| H1 (-500/-408) | gcgaaggaaagttcttcctcgttgttccaatccgaggacaagctgatatgtcgcagcagcccagggaagcatgcgagctgatagg aagtcctt |
| H2 (-403/-351) | tttaagacaggctcgaatgctaaaactttcttgtgccaaaacccttgactatt |
| H3 (-342/-262) | aaaataagcacttggcgtgccctcgcagatgtctgagctgagaggtcggggcgatggtagaagagcagtcagtgtccattc |
| H4 (-181/-140) | tgaatccaaacaggagttgcactagcggtgtccaccacgttg |
| Warm spot region | Sequence of warm spot region |
| W1 (-249/-193) | agtagccaagtctgtacccttgaagcaagtggggagagaggagggagaggagctgct |
| W2 (-123/-89) | ccttctgggagctgtagtttttcgtgggagcggctc |
| W3 (-62/-36) | cgggccgaactacagatcccaggcggc |

**Example 3. saRNA Promoting MITF mRNA Expression in Human Epidermal**

**Melanocytes (HEMs)**

(1) Cell culture and transfection

Cell culture was described in Example 2, human epidermal melanocytes (HEMs) were plated at $20 \times 10^4$ cells/well into a 6-well plate, small activating RNAs were transfected at a final concentration of 25 nM for 72 h, and 2 replicate wells were used for each treatment.

(2) Two-step RT-qPCR

After transfection, the media were discarded, each well was added with 500 $\mu$L of cell lysis solution, and incubation was performed at room temperature for 5 min. RNA was extracted using Qiagen RNeasy kit. After reverse transcription, qPCR analysis on an ABI 7500 fast real-time PCR system (Applied Biosystems), and each sample was repeatedly amplified in 3 replicate wells. PCR reaction conditions are shown in Table 6 and Table 7 below.

Table 6. RT reaction preparation

| Reagent (RT reaction 1) | Volume |
|---|---|
| $5\times$ gDNA Eraser buffer | 2 $\mu$l |
| gDNA Eraser | 1 $\mu$l |
| Total amount of RNA (1 $\mu$g) + D.W | 7 $\mu$l |
| Final volume | 10 $\mu$l |
| 2 min at 42 °C and stored at 4 °C | |
| | |
| Reagent (RT reaction 2) | Volume |
| $5\times$ Prime Script buffer 2 | 4 $\mu$l |
| PrimeScript RT Enzyme Mix 1 | 1 $\mu$l |
| RT primer mixture | 1 $\mu$l |
| No RNase dH$_2$O | 4 $\mu$l |
| RT reaction 1 | 10 $\mu$l |
| Final volume | 20 $\mu$l |
| 15 min at 37 °C, 5 s at 85 °C, stored at 4 °C | |

Table 7. RT-qPCR reaction preparation

| Reagent | Volume |
|---|---|
| SYBR Premix Ex Taq II (2 ×) | 5 μl |
| ROX reference dye II (50×) | 0.2 μl |
| Forward and Reverse Primers Mix (5 μM) | 0.8 μl |
| cDNA (RT products) | 4 μl |
| Sum | 10 μl |

Reaction conditions were as follows: 30 s at 95 °C, 5 s at 95 °C, 30 s at 60 °C, 40 cycles of amplification. At the same time, HPRT1 gene was amplified as an internal reference, and MITF was amplified using the MITF F1/R1 primer pair, the amplification primers are shown in Table 2.

To calculate the expression value (Erel) of MITF (target gene) of a saRNA-transfected sample relative to control treatment (Mock), the Ct values of the target gene and the one internal reference gene were substituted into formula 2 for calculation.

$$E_{rel} = 2^{\text{(CtTm-CtTs)}} / 2^{\text{(CtRm-CtRs)}} \qquad \text{(formula 2)}$$

wherein CtTm was the Ct value of the target gene from the control treatment (Mock) sample; CtTs was the Ct value of the target gene from the saRNA-treated sample; CtRm was the Ct value of the internal reference gene from the control treatment Mock-treated sample; and CtRs was the Ct value of the internal reference gene from the saRNA-treated sample.

FIG. 3 shows relative MITF mRNA expression value of different saRNA-treated cells. The control group (Mock) was used as a blank transfection control, the relative **mRNA** expression value of the siRNA (RAG4-618i) group was knocked down by 81.0% compared to the control group, indicating that the siRNA was successful as a small interfering RNA control transfection. Compared with the control group, the MITF mRNA expression was increased after the cells were treated by different saRNAs, especially the MITF expression was upregulated by 3.9 folds, 3.1 folds and 6.1 folds by RAG4-175, RAG4-176 and RAG4-290, respectively. Other saRNAs (RAG4-284, RAG4-123, RAG4-396, RAG4-461, RAG4-490, RAG4-316 and RAG4-318) increased relative MITF mRNA expression by about 2 folds. This indicated that randomly selected functional saRNAs were capable of being validated for activity in HEM cells.

## Example 4. saRNA Promoting MITF mRNA Expression in NHEK

Cell culture was described in Example 2, normal human epidermal keratinocytes (NHEKs) were plated at 20 × 10⁴ cells/well into a 6-well plate, small activating RNAs were transfected at a final concentration of 25 nM for 72 h, and 2 replicate wells were used for each treatment. Two-step RT-qPCR was as described in Example 3.

FIG. 4 shows relative MITF mRNA expression value of different saRNA-treated cells. The control group (Mock) was used as a blank transfection control, the relative mRNA expression value of the siRNA (RAG4-618i) group decreased by 88.5% compared to the control group, indicating that the siRNA was successful as a small interfering RNA control transfection. Compared with the control group, the siRNA after the treatment of the RAG4-290 had a particularly significant activation effect, and the relative MITF mRNA expression value was increased by 13.1 folds. The relative expression values of mRNA of the RAG4-175, the RAG4-176 and the RAG4-316 groups were higher than those of the control group, the relative expression values of the mRNA in the three groups were increased by 5.7 folds, 3.7 folds and 3.5 folds, respectively, and the siRNAs in the three groups had a significant activation effect. The mRNA relative expression value in the RAG4-284, RAG4-123, RAG4-396, RAG4-461, RAG4-490 and RAG4-318 groups was about 2 folds higher than that in the control group, and the siRNAs in these groups had a certain activation effect. This indicated that randomly selected functional saRNAs were capable of being validated for activity in NHEK cells.

## Example 5. saRNA Promoting MITF Protein Expression in NHEK

Cell culture was described in Example 2, normal human epidermal keratinocytes (NHEKs) were plated at 20 × 10⁴ cells/well into a 6-well plate, and small activating RNAs were transfected at a final concentration of 25 nM for 5 days. An appropriate amount of cell lysis solution containing protease inhibitors (1× RIPA buffer, Cell Signaling Technology) were used for lysis. Protein quantification was performed by using the BCA method, polyacrylamide gel electrophoresis separation was then performed, and the protein was transferred to a 0.45 $\mu$m PVDF membrane. Rabbit monoclonal anti-MITF (Cell Signaling Technology, #12590) $\alpha/\beta$-tubulin antibody (Cell Signaling Technology, 2148s) was used as a primary antibody to detect blots. Anti-rabbit IgG, HRP-linked antibody (Cell Signaling Technology) was used as a secondary antibody. Image Lab (BIO-RAD, Chemistry Doctm MP Imaging System) was used to scan detecting signals.

FIG. 5 shows the relative MITF protein expression level in cells treated with different saRNAs. Compared with the control group, the relative MITF protein expression level in the siRNA (RAG4-618i) group was decreased by about 50%, indicating that siRNA was successful as a small interfering RNA control transfection. Compared with the control group, each saRNA could improve the MITF protein expression level, wherein the MITF protein expression level was regulated by more than 1.5 folds by the RAG4-416, the RAG4-490 and the RAG4-318, particularly the activation effect of the RAG4-318 was most significant, and the MITF protein expression level was increased by 1.71 folds. This indicated that randomly selected functional saRNA can be verified for activity at the protein level.

**Example 6. saRNA Promoting the MITF Protein Expression in Human Epidermal Melanocytes (HEMs)**

Cell culture was described in Example 2, human epidermal melanocytes (HEMs) were plated at $20 \times 10^4$ cells/well into a 6-well plate, and small activating RNAs were transfected at a final concentration of 25 nM for 72 h. Protein lysis and detection methods were as described in Example 5.

FIG. 6 shows the relative MITF protein expression level in cells treated with different saRNAs. Compared with the control group, each saRNA could improve the MITF protein expression level, and the protein expression level in the RAG4-396 and RAG4-490 groups was increased by 1.4 folds. The the MITF protein expression level in each group of RAG4-175, RAG4-176, RAG4-290, RAG4-284 and RAG4-123 was higher than that in the control group, the activation level was above 1.5 folds, and the activation effect was significant. This indicated that randomly selected functional saRNA can be verified for activity at the protein level.

Based on the results above, a plurality of human saRNAs capable of activating the MITF gene expression were found through high-throughput screening of saRNAs targeting human MITF gene promoter. Theses saRNAs can upregulate the MITF gene expression in the cell at mRNA and protein expression level, and can be used for a disease or symptom caused by insufficient or decreased MITF protein expression, such as vitiligo, or for preparing a methods or a drug for treating the disease or symptom.

Various embodiments of the present disclosure have been described above, and the above description is exemplary, not exhaustive, and not limited to the disclosed embodiments. Many modifications and variations will be apparent to those of ordinary skill in the art without departing from the scope and spirit of the described embodiments. The selected terms used herein were to best explain the principles of the various embodiments, practical application, or technical improvements to the market, or to enable others of ordinary skill in the art to understand the various embodiments disclosed herein.

**References**

1. Picardo, M., M. L. Dell'Anna, K. Ezzedine, I. Hamzavi, J. E. Harris, D. Parsad, and A. Taieb. 2015. 'Vitiligo', Nat Rev Dis Primers, 1: 15011.

2. Gauthier, Y, M. Cario Andre, and A. Taieb. 2003. 'A critical appraisal of vitiligo etiologic theories. Is melanocyte loss a melanocytorrhagy?', Pigment Cell Res, 16: 322-32.

3. Dell'anna, M. L., and M. Picardo. 2006. 'A review and a new hypothesis for non-immunological pathogenetic mechanisms in vitiligo', Pigment Cell Res, 19: 406-11.

4. Sehgal, V N., and G. Srivastava. 2007. 'Vitiligo: compendium of clinico-epidemiological features', Indian J Dermatol Venereol Leprol, 73: 149-56.

5. Kotobuki, Y, A. Tanemura, L. Yang, S. Itoi, M. Wataya-Kaneda, H. Murota, M. Fujimoto, S. Serada, T. Naka, and I. Katayama. 2012. 'Dysregulation of melanocyte function by Th17-related cytokines: significance of Th17 cell infiltration in autoimmune vitiligo vulgaris', Pigment Cell Melanoma Res, 25: 219-30.

6. Kemp, E. H., N. G. Gavalas, D. J. Gawkrodger, and A. P. Weetman. 2007. 'Autoantibody responses to melanocytes in the depigmenting skin disease vitiligo', Autoimmun Rev, 6: 138-42.

7. Dell'Anna, M. L., M. Ottaviani, V. Albanesi, A. P. Vidolin, G. Leone, C. Ferraro, A. Cossarizza, L. Rossi, and M. Picardo. 2007. 'Membrane lipid alterations as a possible basis for melanocyte degeneration in vitiligo', J Invest Dermatol, 127: 1226-33.

8. Kumar, R., D. Parsad, and A. J. Kanwar. 2011. 'Role of apoptosis and melanocytorrhagy: a comparative study of melanocyte adhesion in stable and unstable vitiligo', Br J Dermatol, 164: 187-91.

9. Hemesath, T. J., E. Steingrimsson, G. McGill, M. J. Hansen, J. Vaught, C. A. Hodgkinson, H. Arnheiter, N. G. Copeland, N. A. Jenkins, and D. E. Fisher. 1994. 'microphthalmia, a critical factor in melanocyte development, defines a discrete transcription factor family', Genes Dev, 8: 2770-80.

10. Levy, C., M. Khaled, and D. E. Fisher. 2006. 'MITF: master regulator of melanocyte development and melanoma oncogene', Trends Mol Med, 12: 406-14.

11. Kwinter, J., J. Pelletier, A. Khambalia, and E. Pope. 2007. 'High-potency steroid use in children with vitiligo: a retrospective study', JAm Acad Dermatol, 56: 236-41.

12. Iannella, G., A. Greco, D. Didona, B. Didona, G. Granata, A. Manno, B. Pasquariello, and G. Magliulo. 2016. 'Vitiligo: Pathogenesis, clinical variants and treatment approaches', Autoimmun Rev, 15: 335-43.

13. Lim, H. W., P. E. Grimes, O. Agbai, I. Hamzavi, M. Henderson, M. Haddican, R. V. Linkner, and M. Lebwohl. 2015. 'Afamelanotide and narrowband UV-B phototherapy for the treatment of vitiligo: a randomized multicenter trial', JAMA Dermatol, 151: 42-50. 14. Grimes, P. E., I. Hamzavi, M. Lebwohl, J. P. Ortonne, and H. W. Lim. 2013. 'The efficacy of afamelanotide and narrowband UV-B phototherapy for repigmentation of vitiligo', JAMA Dermatol, 149: 68-73.

## Claims

1. A small activating nucleic acid molecule at least comprising a first oligonucleotide strand having at least 75% homology or complementarity to any consecutive fragment of 16 to 35 nucleotides in length in a promoter region of human MITF gene.

2. The small activating nucleic acid molecule according to claim 1, wherein the small activating nucleic acid molecule comprises a first oligonucleotide strand and a second oligonucleotide strand that form a double-stranded structure by complete complementarity or incomplete complementarity.

3. The small activating nucleic acid molecule according to claim 2, wherein the first oligonucleotide strand has at least 75% homology or complementarity to any consecutive fragment of 16 to 35 nucleotides in length in SEQ ID NO: 299, SEQ ID NO: 300, SEQ ID NO: 301, SEQ ID NO: 302, SEQ ID NO: 303, SEQ ID NO: 304 or SEQ ID NO: 305.

4. The small activating nucleic acid molecule according to claim 2, wherein the first oligonucleotide strand has at least 75% homology or complementarity to any nucleotide sequence selected from SEQ ID NOs: 8-104.

5. The small activating nucleic acid molecule according to claim 4, wherein the first oligonucleotide strand comprises any nucleotide sequence selected from SEQ ID NOs: 105-201, or the second oligonucleotide strand comprises any nucleotide sequence selected from SEQ ID NOs: 202-298.

6. The small activating nucleic acid molecule according to any one of claims 2-5, wherein the first oligonucleotide strand has of 16 to 35 nucleotides in length, and the second oligonucleotide strand has of 16 to 35 nucleotides in length.

7. The small activating nucleic acid molecule according to any one of claims 2-6, wherein the small activating nucleic acid molecule is a double-stranded nucleic acid, and the first oligonucleotide strand and the second oligonucleotide strand are located on two strands of the double-stranded nucleic acid.

8. The small activating nucleic acid molecule according to claim 7, wherein the first oligonucleotide strand and/or the second oligonucleotide strand has overhangs at 5' terminus and/or 3' terminus.

9. The small activating nucleic acid molecule according to claim 8, wherein the overhang is an overhang of 0 to 6 nucleotides in length.

10. The small activating nucleic acid molecule according to claim 9, wherein the overhang is dTdT or dTdTdT.

11. The small activating nucleic acid molecule according to any one of claims 2-6, wherein the small activating nucleic acid molecule is a single-stranded nucleic acid having a hairpin structure that can form a double-stranded region, or the first oligonucleotide strand and the second oligonucleotide strand have a complementary region that can form a double-stranded structure.

12. The small activating nucleic acid molecule according to any one of claims 2-11, wherein the first oligonucleotide strand and the second oligonucleotide strand have at least 75% complementarity.

13. The small activating nucleic acid molecule according to any one of claims 1-12, wherein the nucleotide constituting the small activating nucleic acid molecule is a natural and non-chemically modified nucleotide.

14. The small activating nucleic acid molecule according to any one of claims 1-12, wherein one or more nucleotides of the small activating nucleic acid molecule are nucleotides having a chemical modification.

15. The small activating nucleic acid molecule according to claim 14, wherein the chemical modification is one or more selected from the following modifications: modification of phosphodiester bonds of nucleotides, modification of 2'-OH of ribose in nucleotides and modification of base in nucleotides.

16. The small activating nucleic acid molecule according to claim 14, wherein the chemical modification is one or more selected from the following modifications: thiophosphate modification, boranophosphate modification, 2'-fluoro modification, 2'-oxymethyl modification, 2'-oxyethylidene methoxy modification, 2,4'-dinitrophenol modification, locked nucleic acid modification, 2'-amino modification, 2'-deoxy modification, 5'-bromouracil modification, 5'-iodouracil modification, N methyluracil modification and 2,6-diaminopurine modification.

17. The small activating nucleic acid molecule according to any one of claims 14-16, wherein the terminus of the first oligonucleotide strand and/or the second oligonucleotide strand is linked to a lipophilic group, and the lipophilic group is one or more selected from a liposome, a macromolecular polymer, a polypeptide and cholesterol.

18. A nucleic acid molecule comprising a fragment encoding the small activating nucleic acid molecule according to any one of claims 1-13.

19. The nucleic acid molecule according to claim 18, wherein the nucleic acid molecule is an expression vector.

20. A cell comprising the small activating nucleic acid molecule according to any one of claims 1-17 or the nucleic acid molecule according to any one of claims 18-19.

21. A pharmaceutical composition comprising:

the small activating nucleic acid molecule according to any one of claims 1-17 or the nucleic acid molecule according to any one of claims 18-19, and
optionally, a pharmaceutically acceptable carrier.

22. A formulation comprising the small activating nucleic acid molecule according to any one of claims 1-17, or the nucleic acid molecule according to any one of claims 18-19, or the cell according to claim 20, or the pharmaceutical composition according to claim 21.

23. A kit comprising the small activating nucleic acid molecule according to any one of claims 1-17, or the nucleic acid molecule according to any one of claims 18-19, or the cell according to claim 20, or the pharmaceutical composition according to claim 21.

24. Use of the small activating nucleic acid molecule according to any one of claims 1-17, or the nucleic acid molecule according to any one of claims 18-19, or the cell according to claim 20, or the pharmaceutical composition according to claim 21 to prepare a drug or formulation for activating / upregulating the MITF gene expression in a cell.

25. A method for activating / upregulating the MITF gene expression in a cell, wherein the method comprises administering the small activating nucleic acid molecule according to any one of claims 1-17, or the nucleic acid molecule according to any one of claims 18-19, or the composition according to claim 21, or the formulation according to claim 22 to the cell.

26. A method for treating a disease or condition related to insufficient or decreased MITF protein expression, wherein the method comprises administering to an individual in need thereof the small activating nucleic acid molecule according to any one of claims 1-17, or the nucleic acid molecule according to any one of claims 18-19, or the composition according to claim 21, or the formulation according to claim 22.

27. Use of the small activating nucleic acid molecule according to any one of claims 1-17, or the nucleic acid molecule according to any one of claims 18-19, or the cell according to claim 20, or the pharmaceutical composition according to claim 21 to prepare a drug for treating a disease or condition related to insufficient or decreased MITF protein expression.

28. The use according to claim 27, wherein the disease or condition related to insufficient or decreased MITF protein expression is vitiligo, or Waardenburg syndrome type 2A or Tietze syndrome.

FIG. 1

FIG. 2

FIG. 3

FIG. 4

**NHEK (Day5)**

FIG. 5

**HEM (Day3)**

FIG. 6

## INTERNATIONAL SEARCH REPORT

| International application No. |
|---|
| **PCT/CN2021/091834** |

| **A.** | **CLASSIFICATION OF SUBJECT MATTER** |
|---|---|

C12N 15/113(2010.01)i;  A61K 31/713(2006.01)i;  A61P 35/00(2006.01)i

According to International Patent Classification (IPC) or to both national classification and IPC

| **B.** | **FIELDS SEARCHED** |
|---|---|

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

Database: CNABS, VEN (DWPI+SIP0ABS), CNTXT, EPTXT, USTXT, WOTXT, CNKI, PubMed, Elsevier Science, ISI-WEB OF SCIENCE, Genbank, EMBL, 中国专利生物序列检索系统, Chinese Patent Biological Sequence Retrieval System; Search Terms: 小激活核酸, 小激活RNA, 寡核苷酸, 白癜风, 白皮病, 启动子, 小眼畸形相关转录因子, saRNA, RNAa, Vitiligo, leucoderma, promoter, MITF, 序列比对, sequence alignment

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | CN 109071603 A (CAREGEN CO., LTD.) 21 December 2018 (2018-12-21) <br> see entire document | 1-24, 27, 28 |
| A | CN 102429894 A (CHINA PHARMACEUTICAL UNIVERSITY) 02 May 2012 (2012-05-02) <br> see entire document | 1-24, 27, 28 |
| A | CN 110959042 A (SINO-US INSTITUTE OF RNA TECHNOLOGY) 03 April 2020 <br> (2020-04-03) <br> see entire document | 1-24, 27, 28 |
| A | CN 106032532 A (PEKING UNION MEDICAL COLLEGE HOSPITAL, CHINESE <br> ACADEMY OF MEDICAL SCIENCES) 19 October 2016 (2016-10-19) <br> see entire document | 1-24, 27, 28 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 July 2021** | **12 August 2021** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/091834**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

International application No.

**PCT/CN2021/091834**

**Box No. II      Observations where certain claims were found unsearchable (Continuation of item 2 of first sheet)**

This international search report has not been established in respect of certain claims under Article 17(2)(a) for the following reasons:

1. ☑    Claims Nos.: **25-26**
because they relate to subject matter not required to be searched by this Authority, namely:

   [1]   The subject matter of claims 25 and 26 relates to methods of treatment of diseases in the human or animal body.

2. ☐    Claims Nos.:
because they relate to parts of the international application that do not comply with the prescribed requirements to such an extent that no meaningful international search can be carried out, specifically:

3. ☐    Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/091834**

| Patent document cited in search report | | | Publication date (day/month/year) | Patent family member(s) | | | Publication date (day/month/year) |
|---|---|---|---|---|---|---|---|
| CN | 109071603 | A | 21 December 2018 | ES | 2796401 | T3 | 26 November 2020 |
| | | | | EP | 3418292 | A4 | 20 March 2019 |
| | | | | JP | 6672483 | B2 | 25 March 2020 |
| | | | | KR | 20170097832 | A | 29 August 2017 |
| | | | | KR | 101841748 | B1 | 08 May 2018 |
| | | | | EP | 3418292 | A1 | 26 December 2018 |
| | | | | US | 10526373 | B2 | 07 January 2020 |
| | | | | US | 2019119321 | A1 | 25 April 2019 |
| | | | | WO | 2017142264 | A1 | 24 August 2017 |
| | | | | EP | 3418292 | B1 | 08 April 2020 |
| | | | | JP | 2020105198 | A | 09 July 2020 |
| | | | | JP | 2019508488 | A | 28 March 2019 |
| | | | | EP | 3495383 | A1 | 12 June 2019 |
| CN | 102429894 | A | 02 May 2012 | EP | 2789333 | A4 | 03 June 2015 |
| | | | | CA | 2877423 | A1 | 13 June 2013 |
| | | | | EP | 2789333 | A1 | 15 October 2014 |
| | | | | IN | 1411KON2014 | A | 23 October 2015 |
| | | | | CA | 2877423 | C | 01 May 2018 |
| | | | | JP | 2015500249 | A | 05 January 2015 |
| | | | | WO | 2013083040 | A1 | 13 June 2013 |
| | | | | CN | 102429894 | B | 13 March 2013 |
| | | | | US | 2015065580 | A1 | 05 March 2015 |
| | | | | EP | 2789333 | B1 | 15 May 2019 |
| CN | 110959042 | A | 03 April 2020 | CA | 3094575 | A1 | 17 October 2019 |
| | | | | AU | 2019252204 | A1 | 26 November 2020 |
| | | | | WO | 2019196887 | A1 | 17 October 2019 |
| | | | | KR | 20200140377 | A | 15 December 2020 |
| | | | | AU | 2019252204 | A2 | 17 December 2020 |
| | | | | EP | 3778892 | A1 | 17 February 2021 |
| | | | | IL | 277921 | D0 | 30 November 2020 |
| CN | 106032532 | A | 19 October 2016 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SAMBROOK et al.** Molecular Cloning: Laboratory Manual. Cold Spring Harbor Laboratory Press, 1989
- **PICARDO, M. ; M. L. DELL'ANNA ; K. EZZEDINE ; I. HAMZAVI ; J. E. HARRIS ; D. PARSAD ; A. TAIEB.** Vitiligo. *Nat Rev Dis Primers,* 2015, vol. 1, 15011
- **GAUTHIER, Y ; M. CARIO ANDRE ; A. TAIEB.** A critical appraisal of vitiligo etiologic theories. Is melanocyte loss a melanocytorrhagy?. *Pigment Cell Res,* 2003, vol. 16, 322-32
- **DELL'ANNA, M. L. ; M. PICARDO.** A review and a new hypothesis for non-immunological pathogenetic mechanisms in vitiligo. *Pigment Cell Res,* 2006, vol. 19, 406-11
- **SEHGAL, V N. ; G. SRIVASTAVA.** Vitiligo: compendium of clinico-epidemiological features. *Indian J Dermatol Venereol Leprol,* 2007, vol. 73, 149-56
- **KOTOBUKI, Y ; A. TANEMURA ; L. YANG ; S. ITOI ; M. WATAYA-KANEDA ; H. MUROTA ; M. FUJIMOTO ; S. SERADA ; T. NAKA ; I. KATAYAMA.** Dysregulation of melanocyte function by Th17-related cytokines: significance of Th17 cell infiltration in autoimmune vitiligo vulgaris. *Pigment Cell Melanoma Res,* 2012, vol. 25, 219-30
- **KEMP, E. H. ; N. G. GAVALAS ; D. J. GAWKRODGER ; A. P. WEETMAN.** Autoantibody responses to melanocytes in the depigmenting skin disease vitiligo. *Autoimmun Rev,* 2007, vol. 6, 138-42
- **DELL'ANNA, M. L. ; M. OTTAVIANI ; V. ALBANESI ; A. P. VIDOLIN ; G. LEONE ; C. FERRARO ; A. COSSARIZZA ; L. ROSSI ; M. PICARDO.** Membrane lipid alterations as a possible basis for melanocyte degeneration in vitiligo. *J Invest Dermatol,* 2007, vol. 127, 1226-33
- **KUMAR, R. ; D. PARSAD ; A. J. KANWAR.** Role of apoptosis and melanocytorrhagy: a comparative study of melanocyte adhesion in stable and unstable vitiligo. *Br J Dermatol,* 2011, vol. 164, 187-91
- **HEMESATH, T. J. ; E. STEINGRIMSSON ; G. MCGILL ; M. J. HANSEN ; J. VAUGHT ; C. A. HODGKINSON ; H. ARNHEITER ; N. G. COPELAND ; N. A. JENKINS ; D. E. FISHER.** microphthalmia, a critical factor in melanocyte development, defines a discrete transcription factor family. *Genes Dev,* 1994, vol. 8, 2770-80
- **LEVY, C. ; M. KHALED ; D. E. FISHER.** MITF: master regulator of melanocyte development and melanoma oncogene. *Trends Mol Med,* 2006, vol. 12, 406-14
- **KWINTER, J. ; J. PELLETIER ; A. KHAMBALIA ; E. POPE.** High-potency steroid use in children with vitiligo: a retrospective study. *JAm Acad Dermatol,* 2007, vol. 56, 236-41
- **IANNELLA, G. ; A. GRECO ; D. DIDONA ; B. DIDONA ; G. GRANATA ; A. MANNO ; B. PASQUARIELLO ; G. MAGLIULO.** Vitiligo: Pathogenesis, clinical variants and treatment approaches. *Autoimmun Rev,* 2016, vol. 15, 335-43
- **LIM, H. W. ; P. E. GRIMES ; O. AGBAI ; I. HAMZAVI ; M. HENDERSON ; M. HADDICAN ; R. V. LINKNER ; M. LEBWOHL.** Afamelanotide and narrowband UV-B phototherapy for the treatment of vitiligo: a randomized multicenter trial. *JAMA Dermatol,* 2015, vol. 151, 42-50
- **GRIMES, P. E. ; I. HAMZAVI ; M. LEBWOHL ; J. P. ORTONNE ; H. W. LIM.** The efficacy of afamelanotide and narrowband UV-B phototherapy for repigmentation of vitiligo. *JAMA Dermatol,* 2013, vol. 149, 68-73